(19) Europäisches Patentamt
European Patent Office
Office européen des brevets

(11) **EP 1 724 323 A1**

(12) **EUROPEAN PATENT APPLICATION**
published in accordance with Art. 158(3) EPC

(43) Date of publication:
**22.11.2006 Bulletin 2006/47**

(21) Application number: **05720055.2**

(22) Date of filing: **04.03.2005**

(51) Int Cl.:
***C09K 11/06*** *(2006.01)* ***C07D 239/26*** *(2006.01)*
***C07D 401/14*** *(2006.01)* ***C07D 403/10*** *(2006.01)*
***H05B 33/14*** *(2006.01)*

(86) International application number:
**PCT/JP2005/003783**

(87) International publication number:
**WO 2005/085387 (15.09.2005 Gazette 2005/37)**

(84) Designated Contracting States:
**AT BE BG CH CY CZ DE DK EE ES FI FR GB GR HU IE IS IT LI LT LU MC NL PL PT RO SE SI SK TR**

(30) Priority: **08.03.2004 JP 2004064004**

(71) Applicant: **IDEMITSU KOSAN CO., LTD.**
**Tokyo 100-8321 (JP)**

(72) Inventors:
- **IKEDA, Kiyoshi**
  **Sodegaura-shi, Chiba 2990293 (JP)**
- **TOMITA, Seiji**
  **Sodegaura-shi, Chiba 2990293 (JP)**
- **ARAKANE, Takashi**
  **Sodegaura-shi, Chiba 2990293 (JP)**
- **ITO, Mitsunori**
  **Sodegaura-shi, Chiba 2990293 (JP)**

(74) Representative: **Gille Hrabal Struck Neidlein Prop Roos**
**Patentanwälte**
**Brucknerstrasse 20**
**40593 Düsseldorf (DE)**

# (54) MATERIAL FOR ORGANIC ELECTROLUMINESCENCE DEVICE AND ORGANIC ELECTROLUMINESCENCE DEVICE UTILIZING THE SAME

(57) Provided is a material for an organic electroluminescence (EL) device having high luminous efficiency, high thermostability, and a long lifetime, and an organic EL device utilizing the same. The material for an organic EL device is composed of a compound of a specified structure having a nitrogenous ring. The organic EL device has an organic thin film layer composed of one or more layers including at least a light emitting layer, the organic thin film layer being interposed between a cathode and an anode. In the organic EL device, at least one layer of the organic thin film layer contains the material for an organic EL device.

EP 1 724 323 A1

**Description**

TECHNICAL FIELD

The present invention relates to a material for an organic electroluminescence device and an organic electroluminescence device utilizing the same, in particular, a material for an organic electroluminescence device having high luminous efficiency, high thermostability, and a long lifetime and an organic electroluminescence device utilizing the same.

BACKGROUND ART

An organic EL device having an organic light emitting layer interposed between electrodes has been conventionally researched and developed in an intensive manner owing to, for example, the following reasons:

(1) the organic EL device can be easily handled and produced because it is a complete solid-state device;
(2) the organic EL device does not require any light emitting member because it is capable of spontaneously emitting light;
(3) the organic EL device is suitable for a display because it is excellent in visibility; and
(4) the organic EL device facilitates full colorization.

In general, a fluorescent emission phenomenon (luminescence phenomenon) as energy conversion, occurring when a fluorescent molecule in a singlet excited state (which may hereinafter be referred to as the "S1 state") in an organic light emitting medium undergoes radiative transition to a ground state, is used as the mechanism via which such the organic EL device emits light. In addition, a fluorescent molecule in a triplet excited state (which may hereinafter be referred to as the "T1 state") is also assumed in the organic light emitting medium. However, such the fluorescent molecule gradually undergoes non-radiative transition from the triplet excited state to any other state because its radiative transition to a ground state is forbidden transition. As a result, heat energy is released instead of occurrence of fluorescent emission.

The terms "singlet" and "triplet" as used herein each refer to the redundancy of energy determined by the number of combinations of the total spin angular momentum and total orbital angular momentum of the fluorescent molecule. That is, a singlet excited state is defined as an energy state in the case where a single electron is caused to undergo transition from a ground state with no unpaired electron to a higher energy level while the spin state of an electron remains unchanged. In addition, a triplet excited state is defined as an energy state in the case where a single electron is caused to undergo transition to a higher energy level while the spin state of an electron is reversed. Of course, light emission from the triplet excited state thus defined can be observed at an extremely low temperature such as the temperature at which liquid nitrogen liquefies (-196°C). However, the temperature condition is not practical, and the quantity of emitted light is slight.

By the way, the total luminous efficiency of a conventional organic EL device is related to the efficiency (Φrec) with which injected charge carriers (an electron and a hole) recombine with each other and to the probability (Φrad) that a produced exciton causes radiative transition. Therefore, the total luminous efficiency (Φe1) of an organic EL device is represented by the following equation.

$$\Phi e1 = \Phi rec \times 0.25\Phi rad$$

Here, "0.25" of the coefficient for Φrad in the equation is determined on the basis of the assumption that the probability for producing a singlet exciton is 1/4. Therefore, a theoretical upper limit for the luminous efficiency of the organic EL device is 25% even when it is assumed that recombination and the radiation damping of an exciton each occur at a probability factor of 1. As described above, in the conventional organic EL device, no triplet exciton can be substantially utilized, and only a singlet exciton causes radiative transition, so there arises a problem in that an upper limit value of luminous efficiency is low. In view of the foregoing, attempts have been made to cause a fluorescent emission phenomenon to occur even under a room temperature condition through the transfer of energy from a produced triplet exciton to a phosphorescent dopant by utilizing a triplet exciton (triplet excited state) of an organic light emitting material (host material) (see, for example, Non-patent Document 1). To be more specific, it has been reported that a fluorescent emission phenomenon is caused by constituting an organic EL device including an organic light emitting layer constituted by 4,4-N,N-dicarbazolylbiphenyl and an Ir complex as a phosphorescent dopant.

However, the half lifetime of the organic EL device described in Non-patent Document 1 described above is less than 150 hours, so the practicability of the organic EL device is insufficient. There has been proposed, as a measure against the insufficiency, the use of a carbazole derivative having a glass transition temperature of 110°C or higher as a host

material (see, for example, Patent Document 1). However, a half lifetime shown in each example of the patent document is still short, and thermostability merely to allow the device to be stored at 85°C for 200 hours is achieved. Accordingly, it cannot be said that the device has achieved performance sufficient for practical use.

Patent Document 1: WO 01/072927 A
Non Patent Document 1: Jpn. J. Appl. Phys., 38(1999)L1502

DISCLOSURE OF THE INVENTION

PROBLEMS TO BE SOLVED BY THE INVENTION

The present invention has been made with a view to solving the above problems, and an object of the present invention is to provide a material for the organic EL device having high luminous efficiency, high thermostability, and a long lifetime, and an organic electroluminescence device utilizing the same.

The inventors of the present invention have made intensive studies with a view to achieving the above object. As a result, the inventors have found that the use of a compound of a specified structure represented by the following general formula (1) as a material for an organic EL device enables energy to be sufficiently transported to a phosphorescent material because the energy of the compound in a triplet excited state is sufficiently large, so an organic EL device having improved luminous efficiency, improved thermostability, and a long lifetime can be obtained, thereby completing the present invention.

That is, according to the present invention, there is provided a material for an organic EL device containing at least one kind of a compound represented by the following general formula (1) :

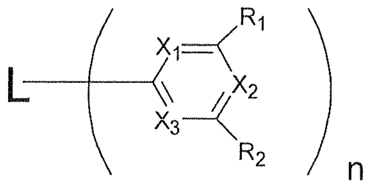

(1)

In the formula, L represents a linking group having at least one meta bond.
$R_1$ and $R_2$ each independently represent a hydrogen atom, an alkyl group which has 1 to 50 carbon atoms and which may have a substituent, a heterocyclic group which has 5 to 50 ring atoms and which may have a substituent, an alkoxy group which has 1 to 50 carbon atoms and which may have a substituent, an aryloxy group which has 5 to 50 ring carbon atoms and which may have a substituent, an aralkyl group which has 7 to 50 ring carbon atoms and which may have a substituent, an alkenyl group which has 2 to 50 carbon atoms and which may have a substituent, an alkylamino group which has 1 to 50 carbon atoms and which may have a substituent, an arylamino group which has 5 to 50 ring carbon atoms and which may have a substituent, an aralkylamino group which has 7 to 50 ring carbon atoms and which may have a substituent, an aryl group which has 6 to 50 ring carbon atoms and which may have a substituent, or a cyano group.
$X_1$ to $X_3$ each independently represent =CR- or =N-, at least one of $X_1$ to $X_3$ represent =N- where R represents an aryl group which has 6 to 50 ring carbon atoms and which may have a substituent, a heterocyclic group which has 5 to 50 ring atoms and which may have a substituent, an alkyl group which has 1 to 50 carbon atoms and which may have a substituent, an alkoxy group which has 1 to 50 carbon atoms and which may have a substituent, an aralkyl group which has 7 to 50 ring carbon atoms and which may have a substituent, an aryloxy group which has 5 to 50 ring carbon atoms and which may have a substituent, an arylthio group which has 5 to 50 ring carbon atoms and which may have a substituent, a carboxyl group, a halogen atom, a cyano group, a nitro group, or a hydroxyl group.
n represents an integer of 1 to 5.
According to the present invention, there is also provided an organic EL device including an organic thin film layer composed of one or more layers including at least a light emitting layer, the organic thin film layer being interposed between a cathode and an anode, in which at least one layer of the organic thin film layer contains the material for an organic EL device.

EFFECT OF THE INVENTION

The organic EL device utilizing a material for an organic EL device of the present invention is extremely practical because the device has high luminous efficiency, high thermostability, and a long lifetime.

BEST MODE FOR CARRYING OUT THE INVENTION

A material for an organic EL device of the present invention is composed of a compound represented by the following general formula (1).

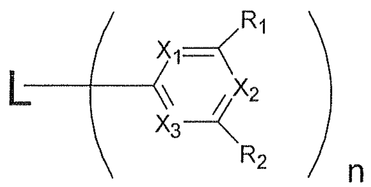

(1)

In the general formula (1), n represents an integer of 1 to 5.
In the general formula(1), $R_1$ and $R_2$ each independently represent a hydrogen atom, an alkyl group which has 1 to 50 carbon atoms and which may have a substituent, a heterocyclic group which has 5 to 50 ring atoms and which may have a substituent, an alkoxy group which has 1 to 50 carbon atoms and which may have a substituent, an aryloxy group which has 5 to 50 ring carbon atoms and which may have a substituent, an aralkyl group which has 7 to 50 ring carbon atoms and which may have a substituent, an alkenyl group which has 2 to 50 carbon atoms and which may have a substituent, an alkylamino group which has 1 to 50 carbon atoms and which may have a substituent, an arylamino group which has 5 to 50 ring carbon atoms and which may have a substituent, an aralkylamino group which has 7 to 50 ring carbon atoms and which may have a substituent, an aryl group which has 6 to 50 ring carbon atoms and which may have a substituent, or a cyano group.
Examples of the alkyl group represented by $R_1$ and $R_2$ include a methyl group, an ethyl group, a propyl group, an isopropyl group, an n-butyl group, an s-butyl group, an isobutyl group, a t-butyl group, an n-pentyl group, an n-hexyl group, an n-heptyl group, an n-octyl group, a hydroxymethyl group, a 1-hydroxyethyl group, a 2-hydroxyethyl group, a 2-hydroxyisobutyl group, a 1,2-dihydroxyethyl group, a 1,3-dihydroxyisopropyl group, a 2,3-dihydroxy-t-butyl group, a 1,2,3-trihydroxypropyl group, a chloromethyl group, a 1-chloroethyl group, a 2-chloroethyl group, a 2-chloroisobutyl group, a 1,2-dichloroethyl group, a 1,3-dichloroisopropyl group, a 2,3-dichloro-t-butyl group, a 1,2,3-trichloropropyl group, a bromomethyl group, a 1-bromoethyl group, a 2-bromoethyl group, a 2-bromoisobutyl group, a 1.,2-dibromoethyl group, a 1,3-dibromoisopropyl group, a 2.,3--dibromo-t-butyl group, a 1,2,3-tribromopropyl group, an iodomethyl group, a 1-iodoethyl group, a 2-iodoethyl group, a 2-iodoisobutyl group, a 1,2-diiodoethyl group, a 1,3-diiodoisopropyl group, a 2,3-diiodo-t-butyl group, a 1,2,3-triiodopropyl group, an aminomethyl group, a 1-aminoethyl group, a 2-aminoethyl group, a 2-aminoisobutyl group, a 1,2-diaminoethyl group, a 1,3-diaminoisopropyl group, a 2,3-diamino-t-butyl group, a 1,2,3-triaminopropyl group, a cyanomethyl group, a 1-cyanoethyl group, a 2-cyanoethyl group, a 2-cyanoisobutyl group, a 1,2-dicyanoethyl group, a 1,3-dicyanoisopropyl group, a 2,3-dicyano-t-butyl group, a 1,2,3-tricyanopropyl group, a nitromethyl group, a 1-nitroethyl group, a 2-nitroethyl group, a 2-nitroisobutyl group, a 1,2-dinitroethyl group, a 1,3-dinitroisopropyl group, a 2,3-dinitro-t-butyl group, a 1,2,3-trinitropropyl group, a cyclopropyl group, a cyclobutyl group, a cyclopentyl group, a cyclohexyl group, a 4-methylcyclohexyl group, a 1-adamantyl group, a 2-adamantyl group, a 1-norbornyl group, a 2-norbornyl group, a cyclopropyl group, a cyclobutyl group, a cyclopentyl group, a cyclohexyl group, and a 4-methylcyclohexyl group.
Examples of the aryl group represented by $R_1$ and $R_2$ include a phenyl group, a 1-naphthyl group, a 2-naphthyl group, a 1-anthryl group, a 2-anthryl group, a 9-anthryl group, a 1-phenanthryl group, a 2-phenanthryl group, a 3-phenanthryl group, a 4-phenanthryl group, a 9-phenanthryl group, a 1-naphthacenyl group, a 2-naphthacenyl group, a 9-naphthacenyl group, a 1-pyrenyl group, a 2-pyrenyl group, a 4-pyrenyl group, a 2-biphenylyl group, a 3-biphenylyl group, a 4-biphenylyl group, a p-terphenyl-4-yl group, a p-terphenyl-4-yl group, a p-terphenyl-3-yl group, a p-terphenyl-2-yl group, an m-terphenyl-4-yl group, a m-terphenyl-3-yl group, a m-terphenyl-2-yl group, an o-tolyl group, an m-tolyl group, a p-tolyl group, a p-t-butylphenyl group, a p-(2-phenylpropyl)phenyl group, a 3-methyl-2-naphthyl group, a 4-methyl-1-naphthyl group, a 4-methyl-1-anthryl group, a 4'-methylbiphenylyl group, and a 4"-t-butyl-p-terphenyl-4-yl group.
Examples of the heterocyclic group represented by $R_1$ and $R_2$ include a 1-pyrolyl group, a 2-pyrolyl group, a 3-pyrolyl group, a pyradinyl group, a 2-pyridinyl group, a 3-pyridinyl group, a 4-pyridinyl group, a 1-indolyl group, a 2-indolyl group, a 3-indolyl group, a 4-indolyl group, a 5-indolyl group, a 6-indolyl group, a 7-indolyl group, a 1-isoindolyl group, a 2-isoindolyl group, a 3-isoindolyl group, a 4-isoindolyl group, a 5-isoindolyl group, a 6-isoindolyl group, a 7-isoindolyl group, a 2-furyl group, a 3-furyl group, a 2-benzofuranyl group, a 3-benzofuranyl group, a 4-benzofuranyl

group, a 5-benzofuranyl group, a 6-benzofuranyl group, a 7-benzofuranyl group, a 1-isobenzofuranyl group, a 3-isobenzofuranyl group, a 4-isobenzofuranyl group, a 5-isobenzofuranyl group, a 6-isobenzofuranyl group, a 7-isobenzofuranyl group, a quinolyl group, a 3-quinolyl group, a 4-quinolyl group, a 5-quinolyl group, a 6-quinolyl group, a 7-quinolyl group, an 8-quinolyl group, a 1-isoquinolyl group, a 3-isoquinolyl group, a 4-isoquinolyl group, a 5-isoquinolyl group, a 6-isoquinolyl group, a 7-isoquinolyl group, an 8-isoquinolyl group, a 2-quinoxalinyl group, a 5-quinoxalinyl group, a 6-quinoxalinyl group, a 1-carbazolyl group, a 2-carbazolyl group, a 3-carbazolyl group, a 4-carbazolyl group, a 9-carbazolyl group, a 1-phenanthridinyl group, a 2-phenanthridinyl group, a 3-phenanthridinyl group, a 4-phenanthridinyl group, a 6-phenanthridinyl group, a 7-phenanthridinyl group, an 8-phenanthridinyl group, a 9-phenanthridinyl group, a 10-phenanthridinyl group, a 1-acridinyl group, a 2-acridinyl group, a 3-acridinyl group, a 4-acridinyl group, a 9-acridinyl group, a 1,7-phenanthrolin-2-yl group, a 1,7-phenanthrolin-3-yl group, a 1,7-phenanthrolin-4-yl group, a 1,7-phenanthrolin-5-yl group, a 1,7-phenanthrolin-6-yl group, a 1,7-phenanthrolin-8-yl group, a 1,7-phenanthrolin-9-yl group, a 1,7-phenanthrolin-10-yl group, a 1,8-phenanthrolin-2-yl group, a 1,8-phenanthrolin-3-yl group, a 1,8-phenanthrolin-4-yl group, a 1,8-phenanthrolin-5-yl group, a 1,8-phenanthrolin-6-yl group, a 1,8-phenanthrolin-7-yl group, a 1,8-phenanthrolin-9-yl group, a 1,8-phenanthrolin-10-yl group, a 1,9-phenanthrolin-2-yl group, a 1,9-phenanthrol-in-3-yl group, a 1,9-phenanthrolin-4-yl group, a 1,9-phenanthrolin-5-yl group, a 1,9-phenanthrolin-6-yl group, a 1,9-phenanthrolin-7-yl group, a 1,9-phenanthrolin-8-yl group, a 1,9-phenanthrolin-10-yl group, a 1,10-phenanthrolin-2-yl group, a 1.,10-phenanthrolin-3-yl group, a 1,10-phenanthrolin-4-yl group, a 1,10-phenanthrolin-5-yl group, a 2,9-phenanthrolin-1-yl group, a 2,9-phenanthrolin-3-yl group, a 2,9-phenanthrolin-4-yl group, a 2,9-phenanthrolin-5-yl group, a 2,9-phenanthrolin-6-yl group, a 2,9-phenanthrolin-7-yl group, a 2,9-phenanthrolin-8-yl group, a 2,9-phenanthrolin-10-yl group, a 2,8-phenanthrolin-1-yl group, a 2,8-phenanthrolin-3-yl group, a 2,8-phenanthrolin-4-yl group, a 2,8-phenanthrolin-5-yl group, a 2,8-phenanthrolin-6-yl group, a 2,8-phenanthrolin-7-yl group, a 2,8-phenanthrolin-9-yl group, a 2,8-phenanthrolin-10-yl group, a 2,7-phenanthrolin-1-yl group, a 2,7-phenanthrolin-3-yl group, a 2,7-phenanthrolin-4-yl group, a 2,7-phenanthrolin-5-yl group, a 2,7-phenanthrolin-6-yl group, a 2,7-phenanthrolin-8-yl group, a 2,7-phenanthrolin-9-yl group, a 2,7-phenanthrolin-10-yl group, a1-phenadinyl group, a 2-phenadinyl group, a1-phenothiadinylgroup, a 2-phenothiadinyl group, a 3-phenothiadinyl group, a 4-phenothiadinyl group, a 10-phenothiadinyl group, a 1-phenoxadinyl group, a 2-phenoxadinyl group, a 3-phenoxadinyl group, a 4-phenoxadinyl group, a 10-phenoxadinyl group, a 2-oxazolyl group, a 4-oxazolyl group, a 5-oxazolyl group, a 2-oxadiazolyl group, a 5-oxadiazolyl group, a 3-furazanyl group, a 2-thienyl group, a 3-thienyl group, a 2-methylpyrrol-1-yl group, a 2-methylpyrrol-3-yl group, a 2-methylpyrrol-4-yl group, a 2-methylpyrrol-5-yl group, a 3-methylpyrrol-1-yl group, a 3-methylpyrrol-2-yl group, a 3-methylpyrrol-4-yl group, a 3-methylpyrrol-5-yl group, a 2-t-butylpyrrol-4-yl group, a 3-(2-phenylpropyl)pyrrol-1-yl group, a 2-methyl-1-indolyl group, a 4-methyl-1-indolyl group, a 2-methyl-3-indolyl group, a 4-methyl-3-indolyl group, a 2-t-butyl-1-indolyl group, a 4-t-butyl-1-indolyl group, a 2-t-butyl-3-indolyl group, and a 4-t-butyl-3-indolyl group.

Further, examples of the heterocyclic group represented by $R_1$ and $R_2$ include: a group in which 1 to 10 benzene rings are bound such as a biphenyl group and a terphenyl group; and a group having a condensed ring such as a naphthyl group, an anthranyl group, a phenanthryl group, a pyrenyl group, and a colonyl group. Of those, a group in which 2 to 5 benzene rings is bound and having many meta bindings which generate torsion of a molecule is particularly preferable.

The alkoxy group represented by $R_1$ and $R_2$ is represented by -OY, and examples of Y are similar to those of the alkyl group.

The aryloxy group represented by $R_1$ and $R_2$ is represented by -OY', and examples of Y' are similar to those of the aryl group.

Examples of the aralkyl group represented by $R_1$ and $R_2$ include a benzyl group, a 1-phenylethyl group, a 2-phenylethyl group, a 1-phenylisopropyl group, a 2-phenylisopropyl group, a phenyl-t-butyl group, an α-naphthylmethyl group, a 1-α-naphthylethyl group, a 2-α-naphthylethyl group, a 1-α-naphthylisopropyl group, a 2-α-naphthylisopropyl group, a β-naphthylmethyl group, a 1-β-naphthylethyl group, a 2-β-naphthylethyl group, a 1-β-naphthylisopropyl group, a 2-β-naphthylisopropyl group, a 1-pyrrolylmethyl group, a 2-(1-pyrrolyl)ethyl group, a p-methylbenzyl group, a m-methylbenzyl group, an o-methylbenzyl group, a p-chlorobenzyl group, a m-chlorobenzyl group, an o-chlorobenzyl group, a p-bromobenzyl group, a m-bromobenzyl group, an o-bromobenzylgroup, a p-iodobenzyl group, a m-iodobenzyl group, an o-iodobenzyl group, a p-hydroxybenzyl group, a m-hydroxybenzyl group, an o-hydroxybenzyl group, a p-aminobenzyl group, a m-aminobenzyl group, an o-aminobenzyl group, a p-nitrobenzyl group, a m-nitrobenzyl group, an o-nitrobenzyl group, a p-cyanobenzyl group, a m-cyanobenzyl group, an o-cyanobenzyl group, a 1-hydroxy-2-phenylisopropyl group, and a 1-chloro-2-phenylisopropyl group.

Examples of the alkenyl group represented by $R_1$ or $R_2$ include a vinyl group, an allyl group, a 1-butenyl group, a 2-butenyl group, a 3-butenyl group, a 1, 3-butanedienyl group, a 1-methylvinyl group, a styryl group, a 2, 2-diphenylvinyl group, a 1,2-diphenylvinyl group, a 1-methylallyl group, a 1,1-dimethylallyl group, a 2-methylallyl group, a 1-phenylallyl group, a 2-phenylallyl group, a 3-phenylallyl group, a 3,3-diphenylallyl group, a 1,2-dimethylallyl group, a 1-phenyl-1-butenyl group, and a 3-phenyl-1-butenyl group.

An example of the alkylamino group, arylamino group, or aralkylamino group represented by $R_1$ or $R_2$ is an amino group substituted by the alkyl group, the aryl group, or the aralkyl group, respectively.

Further, examples of the substituent of each group include a halogen atom, a hydroxyl group, an amino group, a nitro group, a cyano group, an alkyl group, an alkenyl group, a cycloalkyl group, an alkoxy group, an aryl group, a heterocyclic group, an aralkyl group, an aryloxy group, an alkoxycarbonyl group, and a carboxyl group.

In the general formula (1), $X_1$ to $X_3$ each independently represent =CR- or =N- where R represents an aryl group which has 6 to 50 ring carbon atoms and which may have a substituent, a heterocyclic group which has 5 to 50 ring atoms and which may have a substituent, an alkyl group which has 1 to 50 carbon atoms and which may have a substituent, an alkoxy group which has 1 to 50 carbon atoms and which may have a substituent, an aralkyl group which has 7 to 50 ring carbon atoms and which may have a substituent, an aryloxy group which has 5 to 50 ring carbon atoms and which may have a substituent, an arylthio group which has 5 to 50 ring carbon atoms and which may have a substituent, a carboxyl group, a halogen atom, a cyano group, a nitro group, or a hydroxyl group, and at least one of $X_1$ to $X_3$ represents =N-.

Examples of the aryl group, heterocyclic group, alkyl group, alkoxy group, aralkyl group, or aryloxy group represented by R are similar to those described for $R_1$ and $R_2$.

The arylthio group represented by R is represented by -SY', and examples of Y' are similar to those of the aryl group.

Examples of the halogen atom represented by R include fluorine, chlorine, bromine, and iodine.

In the general formula (1), an aromatic heterocyclic compound having one or more hetero atoms in any one of its molecules is preferably used as a nitrogenous ring. Specific compounds of the nitrogenous ring derivative include pyridine, pyrimidine, pyrazine, pyridazine, and triazine.

In the general formula (1), L represents a linking group having at least one meta bond.

In addition, in the general formula (1), L preferably represents a group represented by the following general formula (2), (9), or (10).

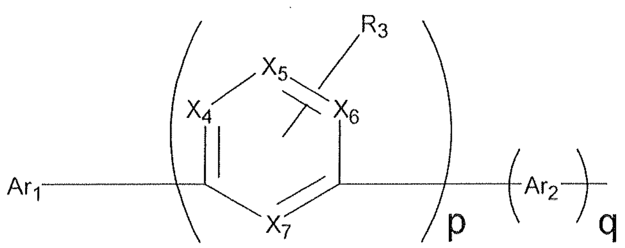

(2)

In the general formula (2), p represents an integer of 1 to 20, and q represents an integer of 1 to 20.

In the general formula (2), $X_4$ to $X_7$ each independently represent =CR- or =N- where R represents any one of the same groups as those described above.

In the general formula (2), $R_3$ represents a hydrogen atom, an alkyl group which has 1 to 50 carbon atoms and which may have a substituent, a heterocyclic group which has 5 to 50 ring atoms and which may have a substituent, an alkoxy group which has 1 to 50 carbon atoms and which may have a substituent, an aryloxy group which has 5 to 50 ring carbon atoms and which may have a substituent, an aralkyl group which has 7 to 50 ring carbon atoms and which may have a substituent, an alkenyl group which has 2 to 50 carbon atoms and which may have a substituent, an alkylamino group which has 1 to 50 carbon atoms and which may have a substituent, an arylamino group which has 5 to 50 ring carbon atoms and which may have a substituent, an aralkylamino group which has 7 to 50 ring carbon atoms and which may have a substituent, an aryl group which has 6 to 50 ring carbon atoms and which may have a substituent, or a cyano group, and two or more $R_3$s may be included.

Examples of the alkyl group, heterocyclic group, alkoxy group, aryloxy group, aralkyl group, alkenyl group, alkylamino group, arylamino group, aralkylamino group, or aryl group represented by $R_3$ are similar to those described for $R_1$ and $R_2$ of the general formula (1).

In the general formula (2), $Ar_2$ represents a heterocyclic group which has 5 to 50 ring atoms and which may have a substituent, an aryleneoxy group which has 5 to 50 ring carbon atoms and which may have a substituent, an aryleneamino group which has 5 to 50 ring carbon atoms and which may have a substituent, or an arylene group which has 6 to 50 ring carbon atoms and which may have a substituent.

Examples of a divalent heterocyclic group, divalent aryleneoxy group, divalent aryleneamino group, or divalent arylene group represented by $Ar_2$ include divalent groups each obtained by removing one hydrogen atom from each of the aryloxy group, the arylamino group, and the aryl group described for $R_1$ and $R_2$ of the general formula (1).

In the general formula (2) , $Ar_1$ represents a heterocyclic group which has 5 to 50 ring atoms and which may have a substituent, an aryloxy group or aryleneoxy group which has 5 to 50 ring carbon atoms and which may have a substituent,

an arylamino group or aryleneamino group which has 5 to 50 ring carbon atoms and which may have a substituent, or an aryl group or arylene group which has 6 to 50 ring carbon atoms and which may have a substituent.
Examples of the heterocyclic group, aryloxy group, arylamino group, or aryl group represented by $Ar_1$ are similar to those described for $R_1$ and $R_2$ of the general formula (1). In addition, examples of a divalent heterocyclic group, divalent aryleneoxy group, divalent aryleneamino group, or divalent arylene group include divalent groups each obtained by removing one hydrogen atom from each of the aryloxy group, the arylamino group, and the aryl group.

In addition, $Ar_1$ described above preferably has a substituent represented by any one of the following general formulae (3) to (8).

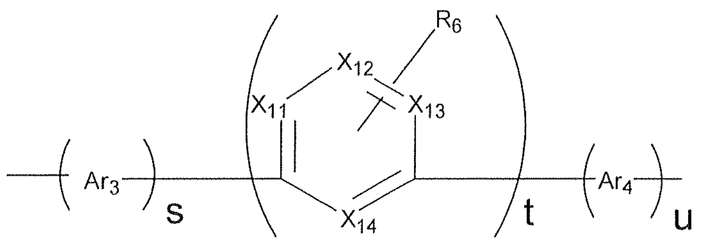

(9)

In the general formula (9), s represents an integer of 0 to 20, t represents an integer of 1 to 20, and u represents an integer of 0 to 20.
In the general formula (9), $X_{11}$ to $X_{14}$ each independently represent =CR- or =N- where R represents any one of the same groups as those described above.
In the general formula (9), $R_6$ represents a hydrogen atom, an alkyl group which has 1 to 50 carbon atoms and which may have a substituent, a heterocyclic group which has 5 to 50 ring atoms and which may have a substituent, an alkoxy group which has 1 to 50 carbon atoms and which may have a substituent, an aryloxy group which has 5 to 50 ring carbon atoms and which may have a substituent, an aralkyl group which has 7 to 50 ring carbon atoms and which may have a substituent, an alkenyl group which has 2 to 50 carbon atoms and which may have a substituent, an alkylamino group which has 1 to 50 carbon atoms and which may have a substituent, an arylamino group which has 5 to 50 ring carbon atoms and which may have a substituent, an aralkylamino group which has 7 to 50 ring carbon atoms and which may have a substituent, an aryl group which has 6 to 50 ring carbon atoms and which may have a substituent, or a cyano group. Two or more $R_6$s may be included.
Examples of the alkyl group, heterocyclic group, alkoxy group, aryloxy group, aralkyl group, alkenyl group, alkylamino group, arylamino group, aralkylamino group, or aryl group represented by $R_6$ are similar to those described for $R_1$ and $R_2$ of the general formula (1).

In the general formula (9), $Ar_3$ and $Ar_4$ each independently represent a heterocyclic group which has 5 to 50 ring atoms and which may have a substituent, an aryleneoxy group which has 5 to 50 ring carbon atoms and which may have a substituent, an aryleneamino group which has 5 to 50 ring carbon atoms and which may have a substituent, or an arylene group which has 6 to 50 ring carbon atoms and which may have a substituent.
Examples of a divalent heterocyclic group, divalent aryleneoxy group, divalent aryleneamino group, or divalent arylene group represented by $Ar_3$ or $Ar_4$ include divalent groups each obtained by removing one hydrogen atom from each of the aryloxy group, the arylamino group, and the aryl group described for $R_1$ and $R_2$ of the general formula (1).
In addition, a compound represented by the general formula (9) preferably has at least one substituent represented by any one of the following general formulae (3) to (8).

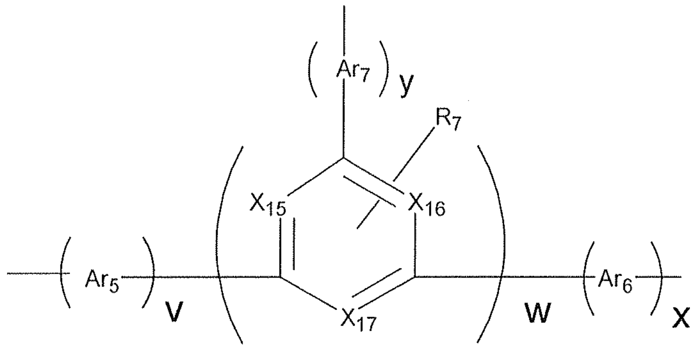

(10)

In the general formula (10), v represents an integer of 0 to 20, w represents an integer of 1 to 20, x represents an integer of 0 to 20, and y represents an integer of 0 to 20.

In the general formula (10), $X_{15}$ to $X_{17}$ each independently represent =CR- or =N- where R represents any one of the same groups as those described above.

In the general formula (10) , $R_7$ represents a hydrogen atom, an alkyl group which has 1 to 50 carbon atoms and which may have a substituent, a heterocyclic group which has 5 to 50 ring atoms and which may have a substituent, an alkoxy group which has 1 to 50 carbon atoms and which may have a substituent, an aryloxy group which has 5 to 50 ring carbon atoms and which may have a substituent, an aralkyl group which has 7 to 50 ring carbon atoms and which may have a substituent, an alkenyl group which has 2 to 50 carbon atoms and which may have a substituent, an alkylamino group which has 1 to 50 carbon atoms and which may have a substituent, an arylamino group which has 5 to 50 ring carbon atoms and which may have a substituent, an aralkylamino group which has 7 to 50 ring carbon atoms and which may have a substituent, an aryl group which has 6 to 50 ring carbon atoms and which may have a substituent, or a cyano group. Two or more $R_7$s may be included.

Examples of the alkyl group, heterocyclic group, alkoxy group, aryloxy group, aralkyl group, alkenyl group, alkylamino group, arylamino group, aralkylamino group, or aryl group represented by $R_7$ are similar to those described for $R_1$ and $R_2$ of the general formula (1).

In the general formula (10) , $Ar_5$ to$Ar_7$ each independently represent a heterocyclic group which has 5 to 50 ring atoms and which may have a substituent, an aryleneoxy group which has 5 to 50 ring carbon atoms and which may have a substituent, an aryleneamino group which has 5 to 50 ring carbon atoms and which may have a substituent, or an arylene group which has 6 to 50 ring carbon atoms and which may have a substituent.

Examples of a divalent heterocyclic group, divalent aryleneoxy group, divalent aryleneamino group, or divalent arylene group represented by $Ar_5$ to $Ar_7$ include divalent groups each obtained by removing one hydrogen atom from each of the aryloxy group, the arylamino group, and the aryl group described for $R_1$ and $R_2$ of the general formula (1).

In addition, a compound represented by the general formula (10) preferably has at least one substituent represented by any one of the following general formulae (3) to (8).

Examples of the structure of L in the general formula (1) include the following structures.

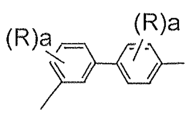
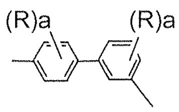
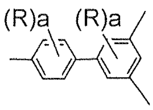
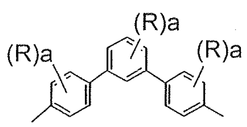

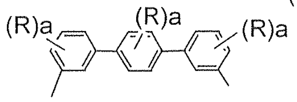
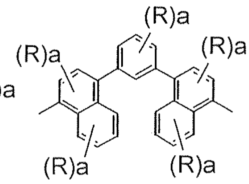
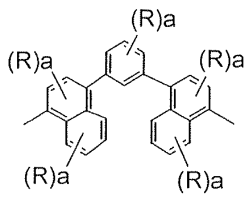
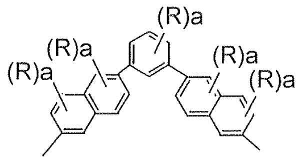

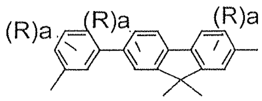
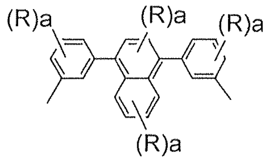
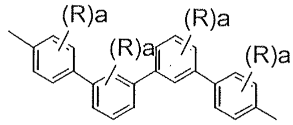

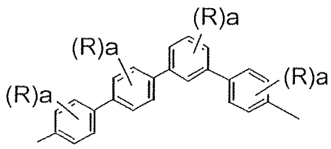
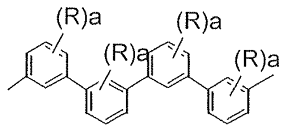
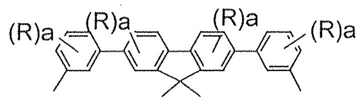

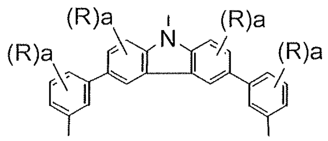
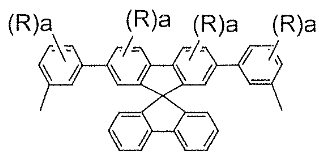
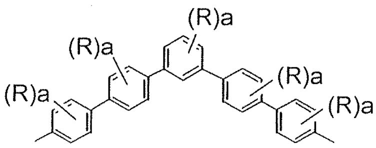

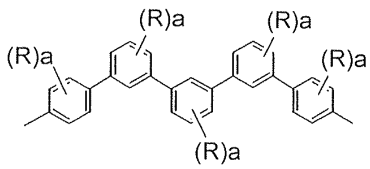
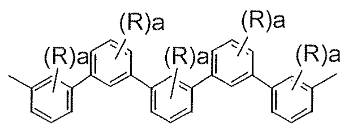
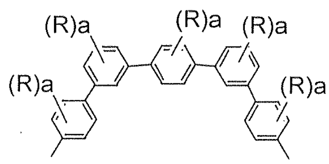

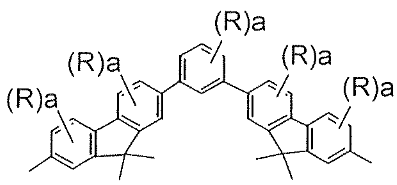
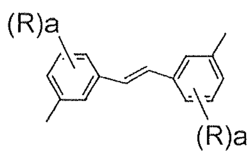

A benzene ring in each of the above formulae may be replaced with a heterocyclic ring such as pyridine, pyrimidine, or triazine.

In each of the above formulae, as in the case of the foregoing, R represents an aryl group which has 6 to 50 ring carbon atoms and which may have a substituent, a heterocyclic group which has 5 to 50 ring atoms and which may have a substituent, an alkyl group which has 1 to 50 carbon atoms and which may have a substituent, an alkoxy group which has 1 to 50 carbon atoms and which may have a substituent, an aralkyl group which has 7 to 50 ring carbon atoms and which may have a substituent, an aryloxy group which has 5 to 50 ring carbon atoms and which may have a substituent, an arylthio group which has 5 to 50 ring carbon atoms and which may have a substituent, a carboxyl group, a halogen

atom, a cyano group, a nitro group, or a hydroxyl group, and a represents an integer of 0 to 4.
In addition, the aryl group which has 6 to 50 carbon atoms may be additionally substituted by a substituent. Examples of a preferable substituent include: carbazolyl groups each represented by any one of the following general formulae (3) to (8); alkyl groups each having 1 to 6 carbon atoms (such as an ethyl group, a methyl group, an i-propyl group, an n-propyl group, an s-butyl group, a t-butyl group, a pentyl group, a hexyl group, a cyclopentyl group, and a cyclohexyl group); alkoxy groups each having 1 to 6 carbon atoms (such as an ethoxy group, a methoxy group, an i-propoxy group, an n-propoxy group, an s-butoxy group, a t-butoxy group, a pentoxy group, a hexyloxy group, a cyclopentoxy group, and a cyclohexyloxy group); aryl groups each having 5 to 50 ring atoms; amino groups each substituted by an aryl group having 5 to 50 ring atoms; ester groups each having an aryl group having 5 to 50 ring atoms; ester groups each having an alkyl group having 1 to 6 carbon atoms; a cyano group; a nitro group; and a halogen atom.
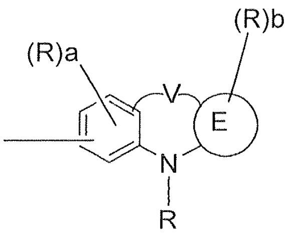 ···(3) 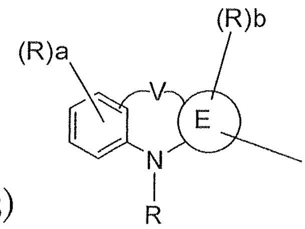 ···(4) 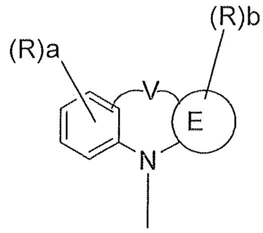 ···(5)

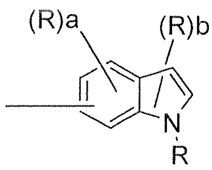 ···(6) 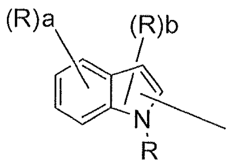 ···(7) 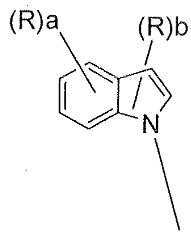 ···(8)

In the general formulae (3) to (8), a and b each represent an integer of 0 to 4.
In the general formulae (3) to (8), R represents any one of the same groups as those described above, and when two or more Rs are included, they may bond to each other to form a ring structure.
In the general formulae (3) to (8) , V represents a single bond, $-CR_0R_0'$ -, $-SiR_0R_0'$-, -O-, -CO-, or $-NR_0$ where $R_0$ and $R_0'$ each independently represent a hydrogen atom, an aryl group which has 6 to 50 ring carbon atoms and which may have a substituent, a heterocyclic group which has 5 to 50 ring atoms and which may have a substituent, or an alkyl group which has 1 to 50 carbon atoms and which may have a substituent.
Examples of the aryl group, heterocyclic group, or alkyl group represented by $R_0$ or $R_0'$ are similar to those described for $R_1$ and $R_2$ of the general formula (1) .
In the general formulae (3) to (8), E represents a cyclic structure represented by a circle surrounding the symbol E, and represents a cycloalkane residue which has 3 to 20 ring carbon atoms and which may have a substituent, and a carbon atom of which may be substituted by a nitrogen atom, an aromatic hydrocarbon residue which has 4 to 50 ring carbon atoms and which may have a substituent, or a heterocyclic residue which has 4 to 50 ring atoms and which may have a substituent.
Specific examples of the aromatic hydrocarbon residue and the heterocyclic residue each represented by E include divalent residues selected from the aryl groups and the heterocyclic groups described for $R_1$ and $R_2$ of the general formula (1) , the carbon number of each of which is adaptable to that of E. In addition, examples of the cycloalkane residue which has 3 to 20 ring carbon atoms and a carbon atom of which may be substituted by a nitrogen atom include divalent residues of cyclopropane, cyclobutane, cyclopropane, cyclohexane, cycloheptane, pyrrolidine, piperidine, piperazine, and the like.
Examples of the general formula (3) include structures represented by the following general formulae (11) to (14) (the same structures can be exemplified for the general formula (4)):

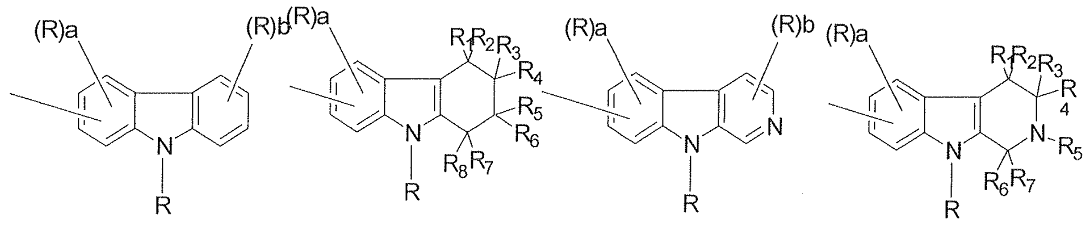

(11) (12) (13) (14)

where a, b, R, and $R_1$ to $R_8$ each have the same meaning as that described above.

Further, specific examples of the general formulae (11) to (14) include the following structures.

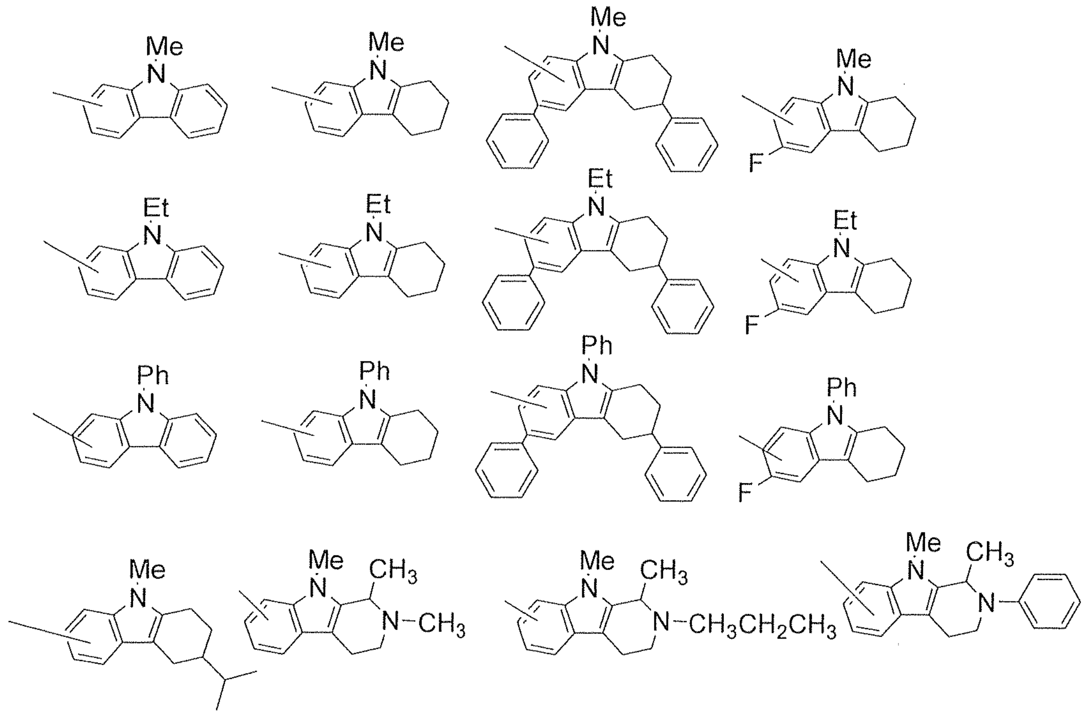

Examples of the general formula (5) include structures represented by the following general formulae (15) to (18):

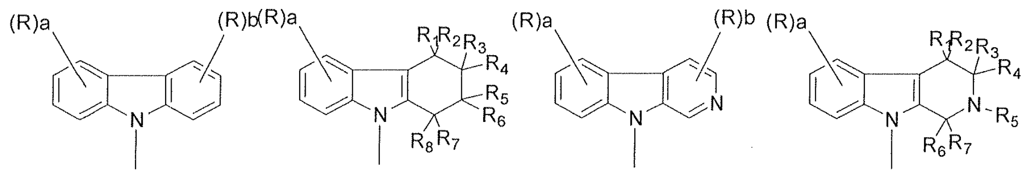

(15) (16) (17) (18)

where a, b, R, and $R_1$ to $R_8$ each have the same meaning as that described above.

Further, specific examples of the general formulae (15) to (18) include the following structures.

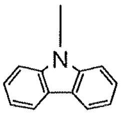 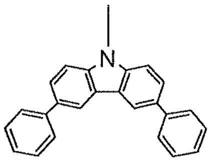 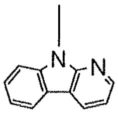 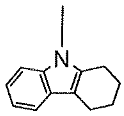 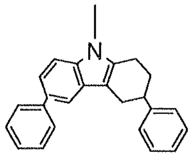 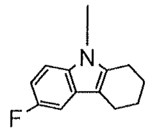

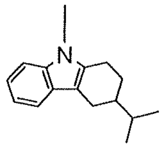 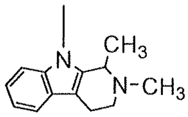 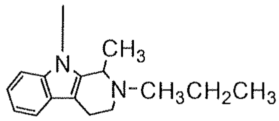 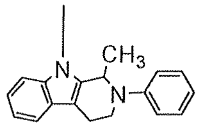

Specific examples of the general formula (6) include the following structures (the same structures can be exemplified for the general formula (7)).

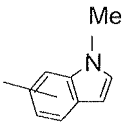 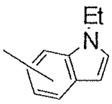 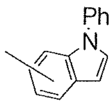 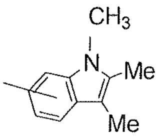 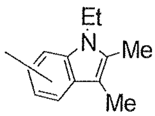 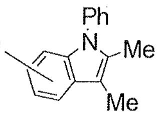

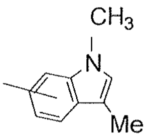 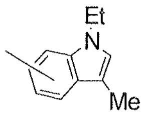 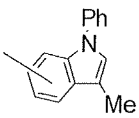 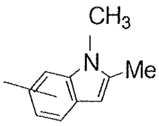 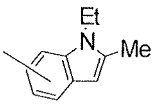 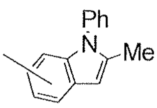

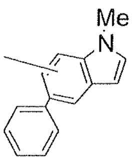 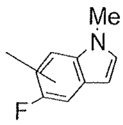

Specific examples of the general formula (8) include the following structures.

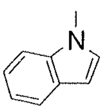 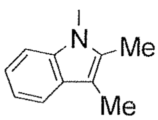 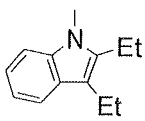 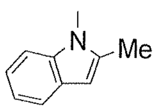 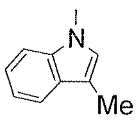

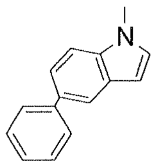
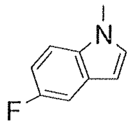

Specific examples of the compound represented by the general formula (1) of the present invention are shown below. However, the examples are not limited to these exemplified compounds.

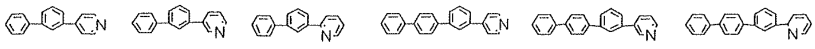
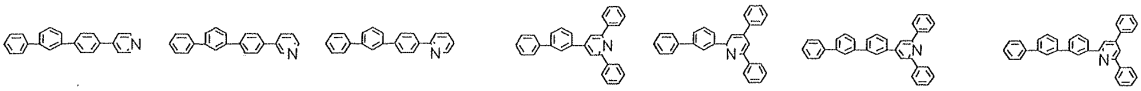
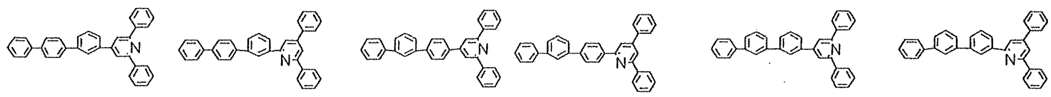
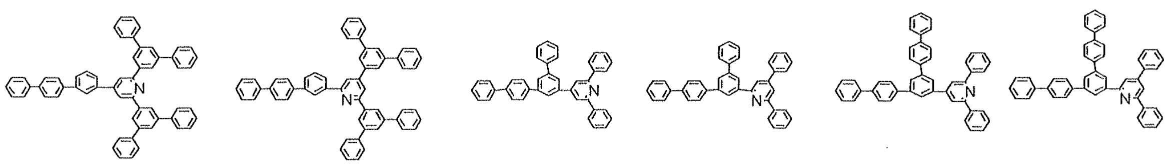
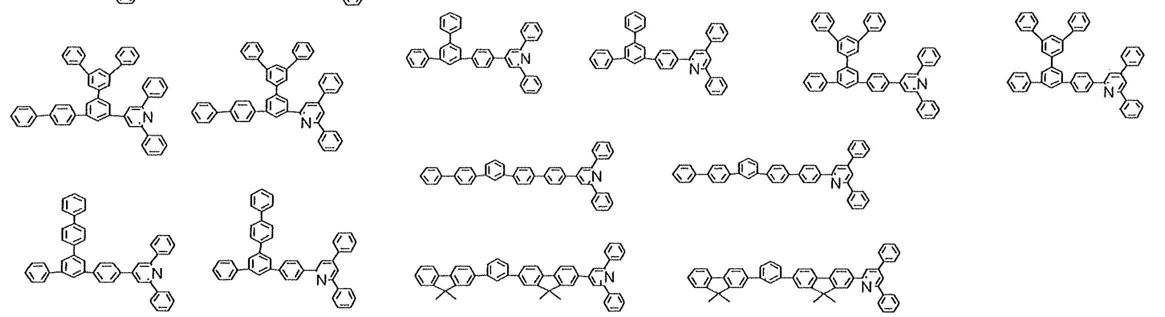
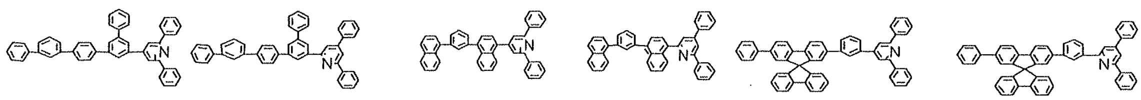
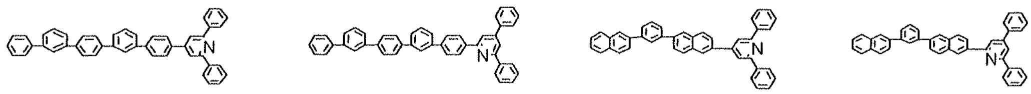
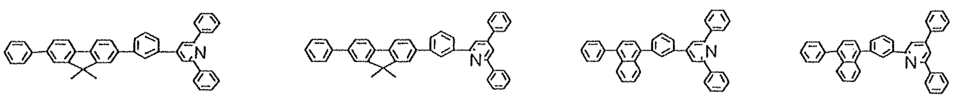

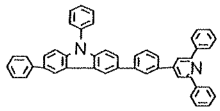
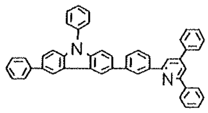
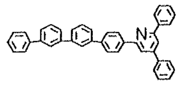
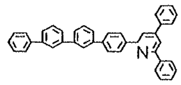

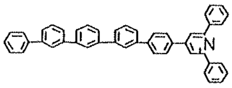
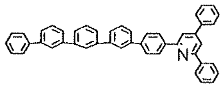
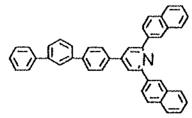
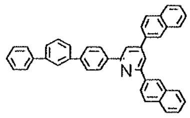

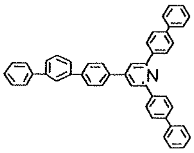
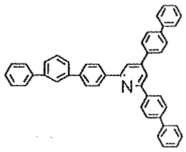
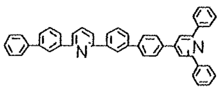
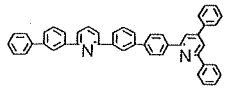

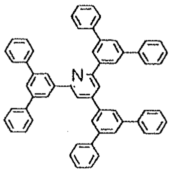
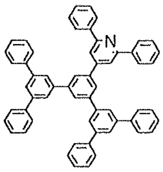

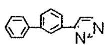 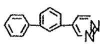 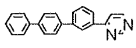 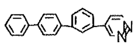 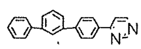 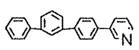

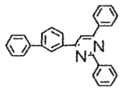 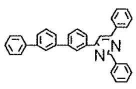 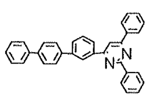 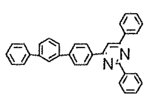 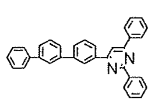

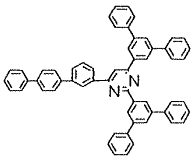 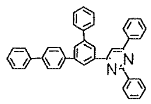 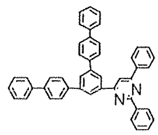 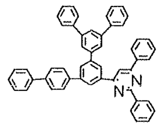 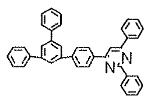 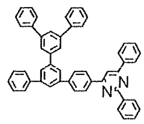

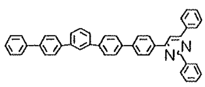 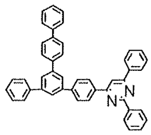 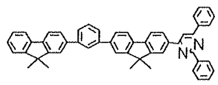 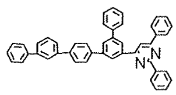 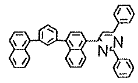

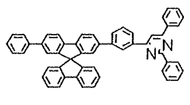 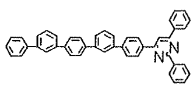 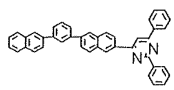 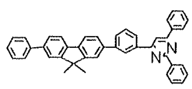 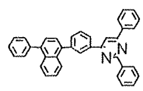

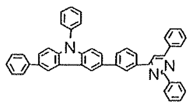 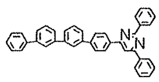 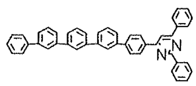 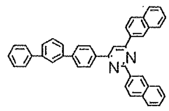 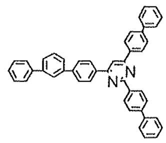

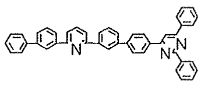 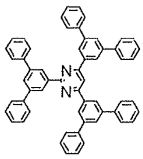 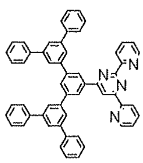

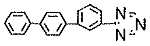 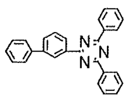 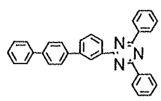 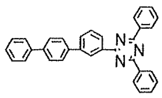

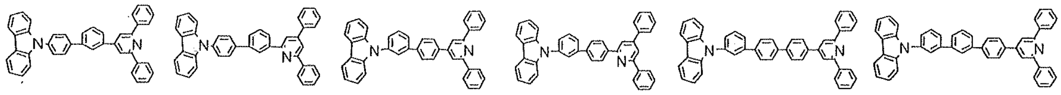
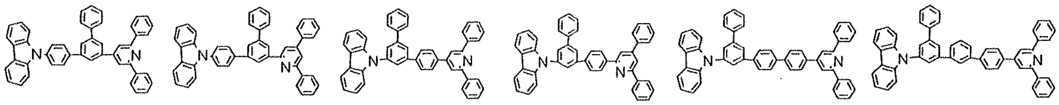
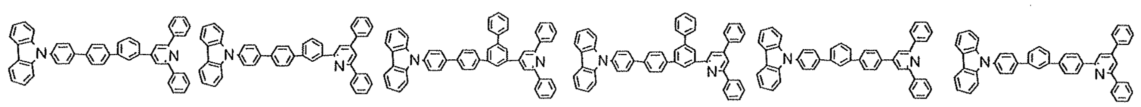
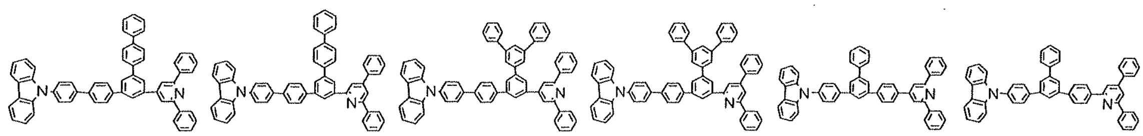
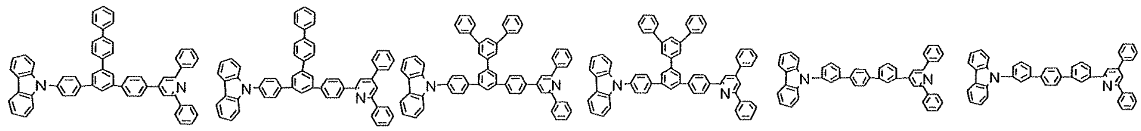
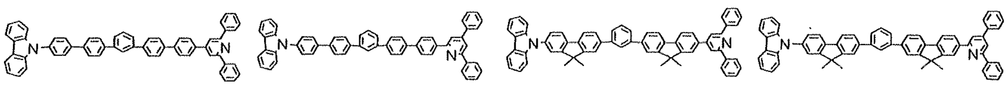
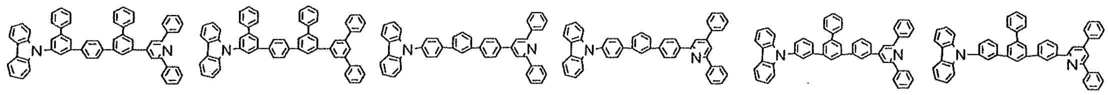
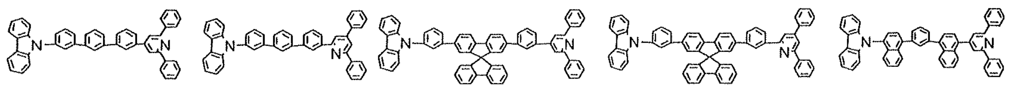
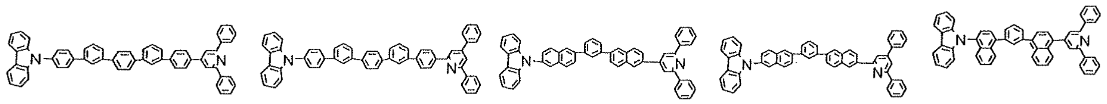
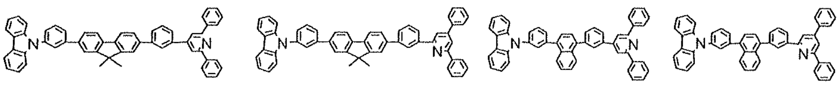
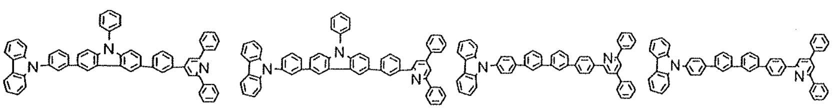
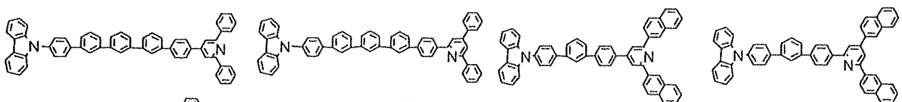
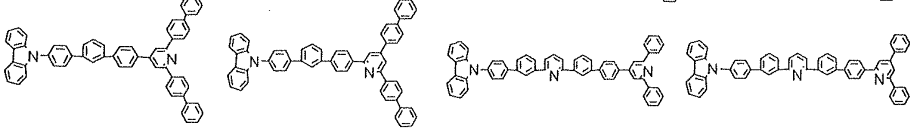

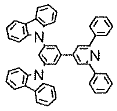
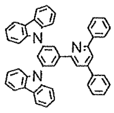
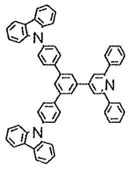
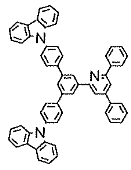
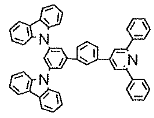
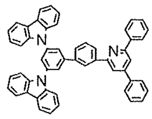

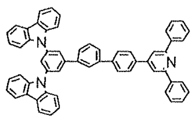
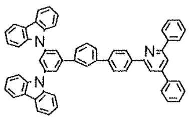
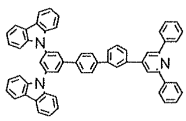
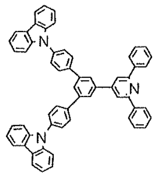
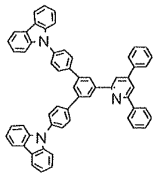

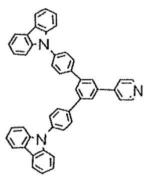
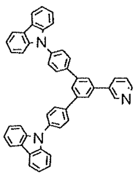
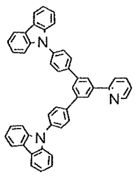
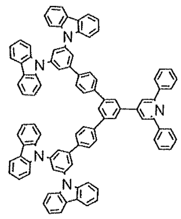
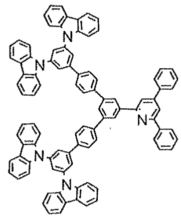

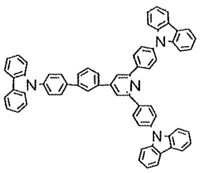
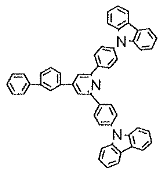
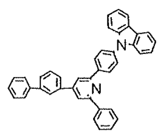
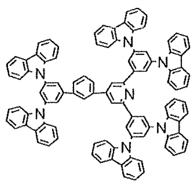
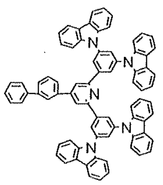
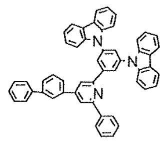

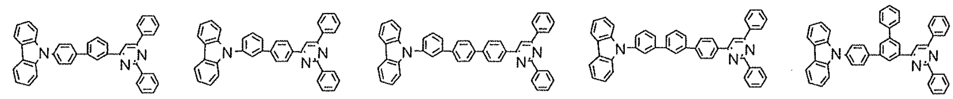

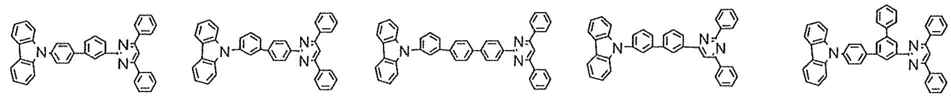

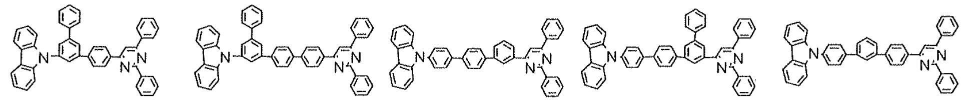

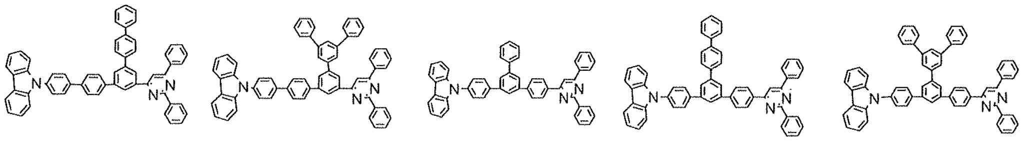

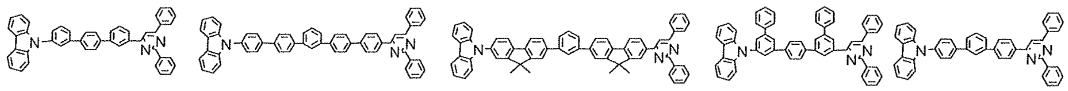

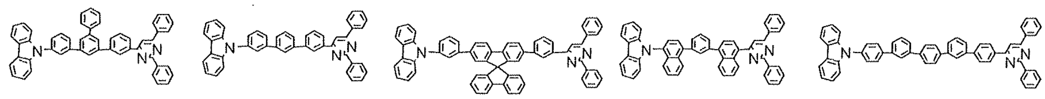

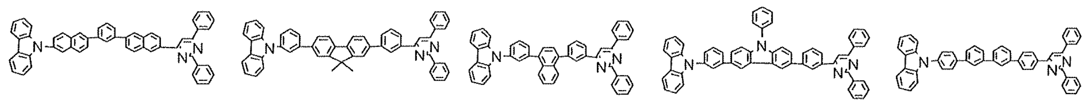

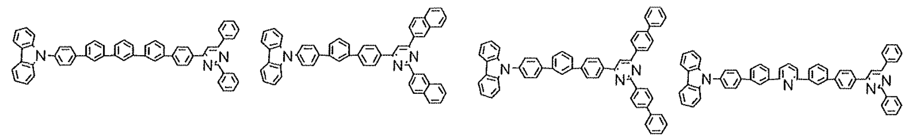

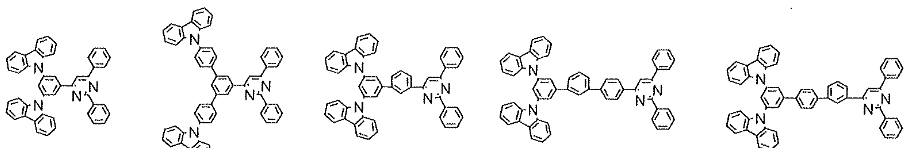

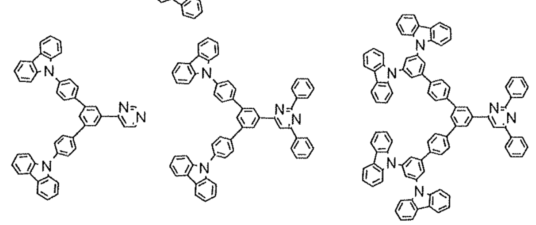

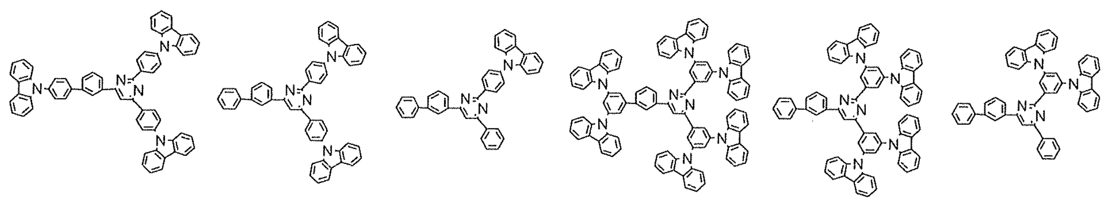

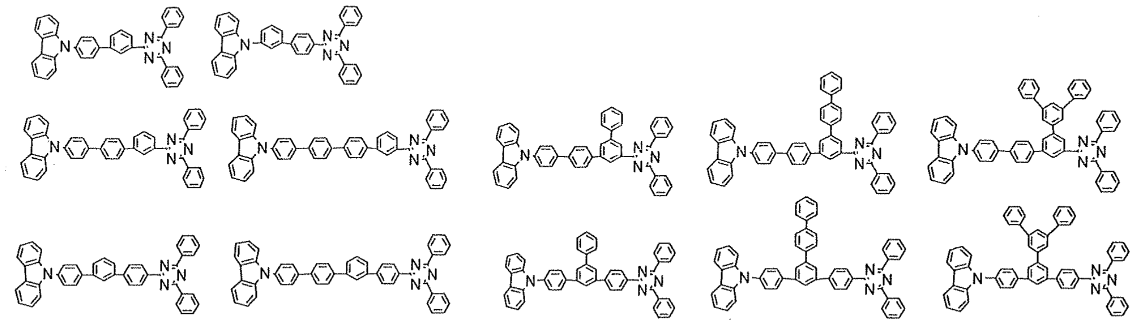

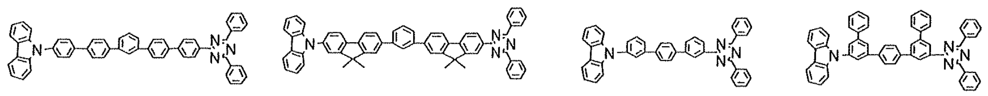

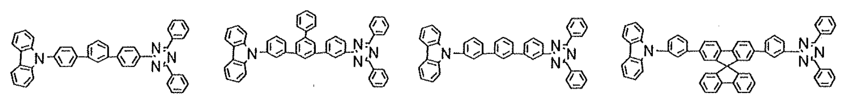

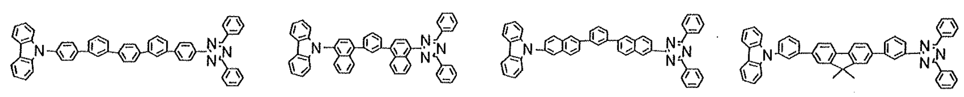

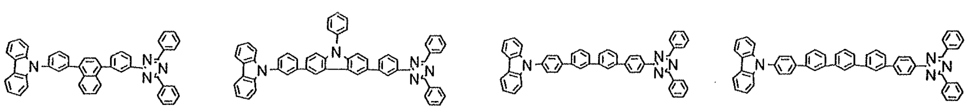

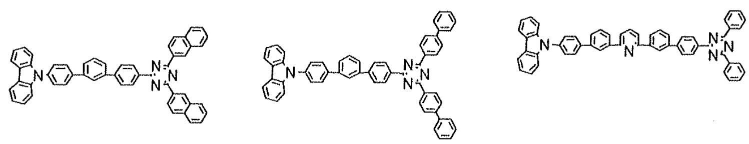

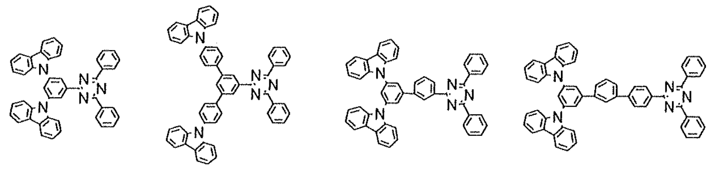

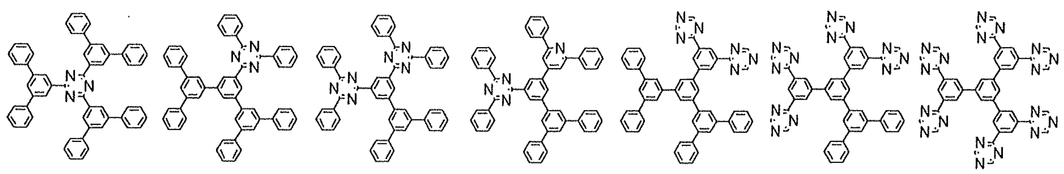

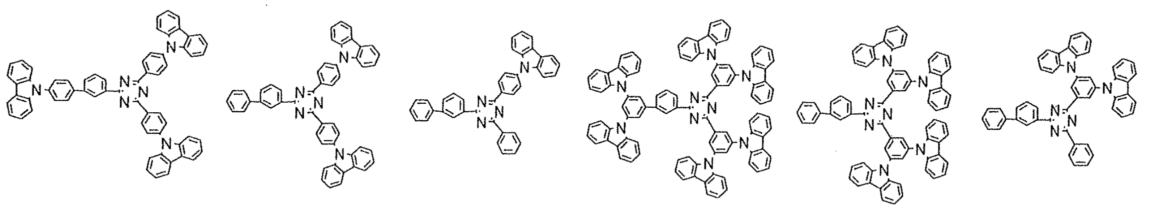

36

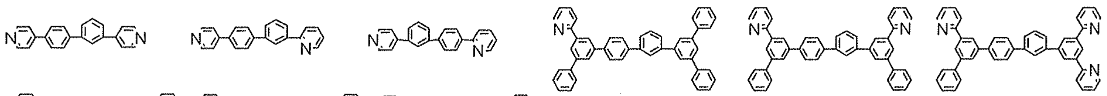
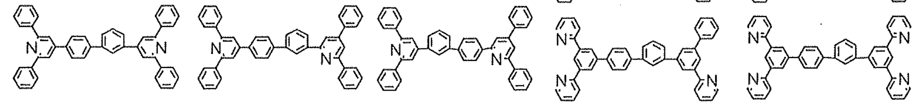
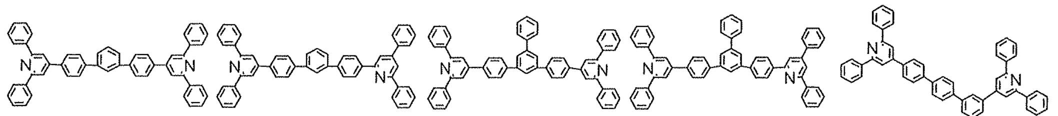
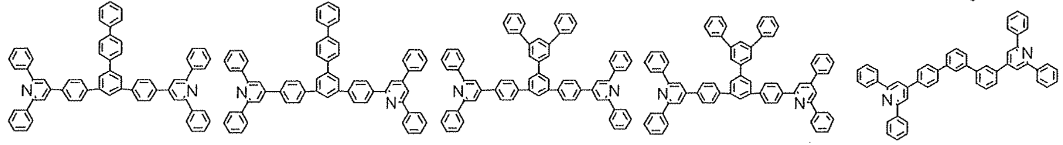
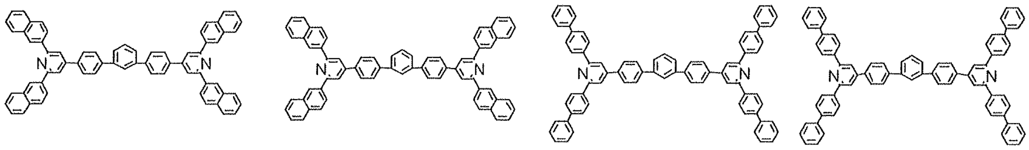
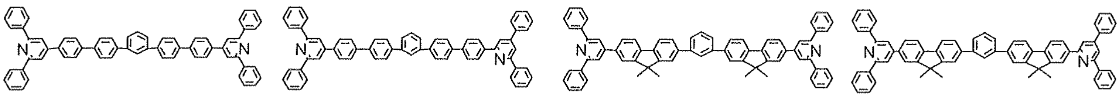
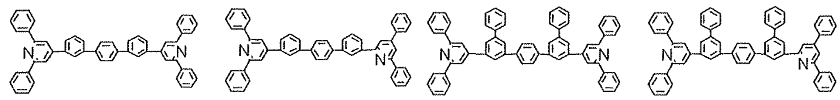
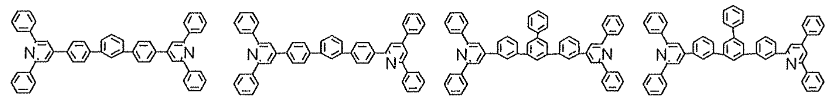
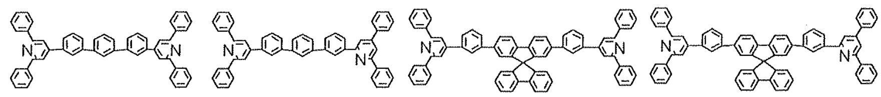
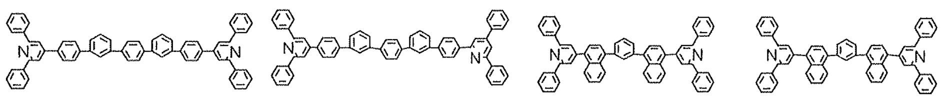
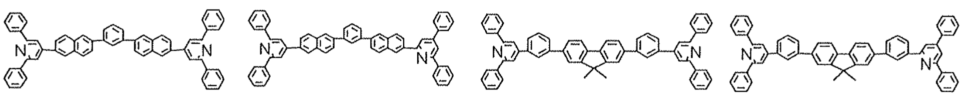
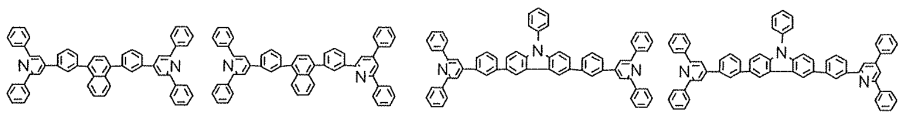
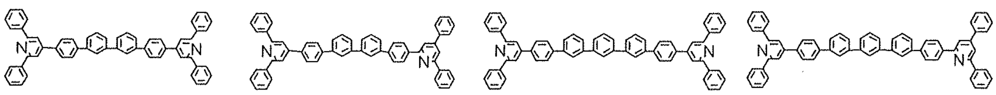
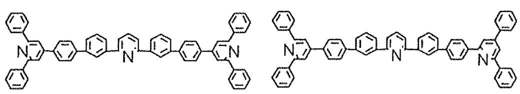

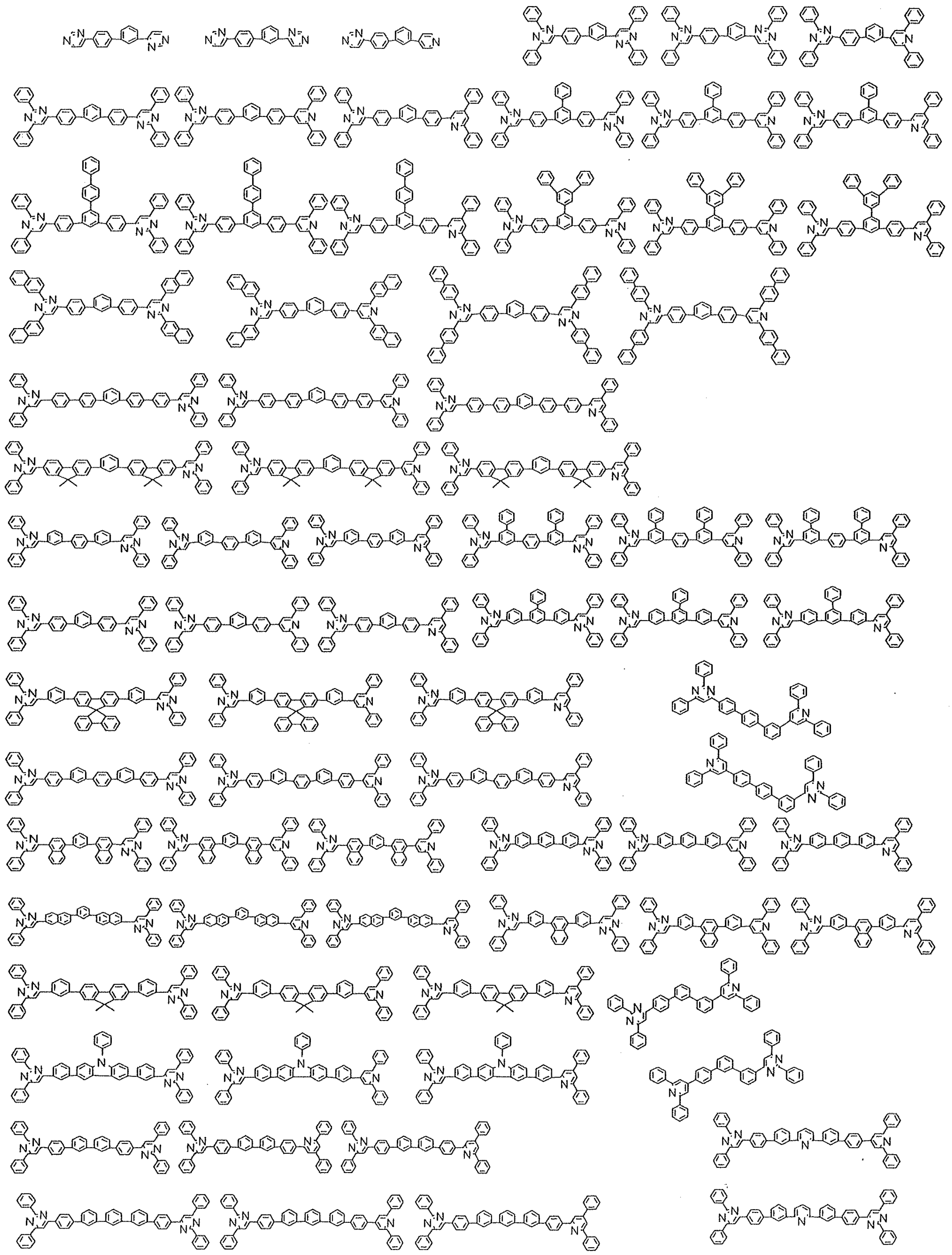

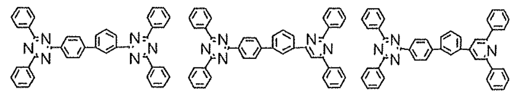
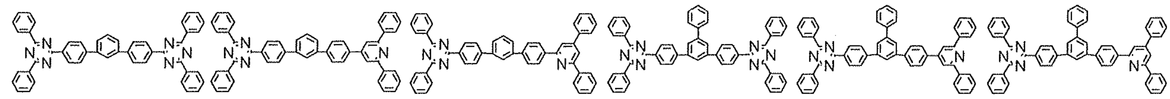
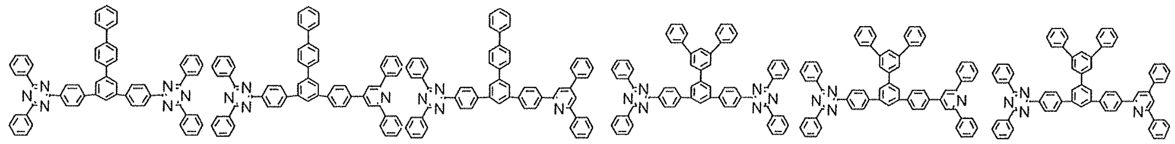
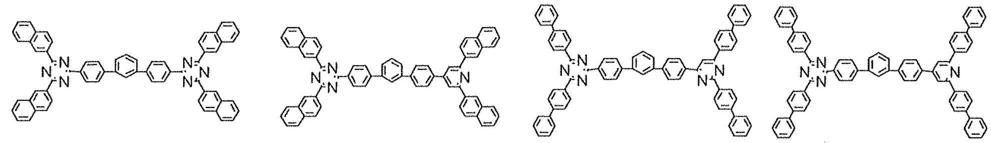
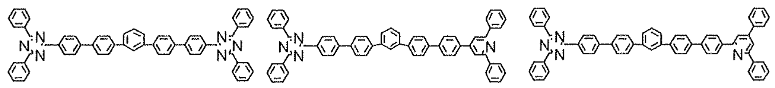
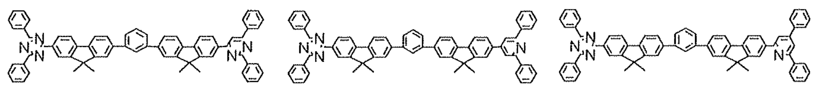
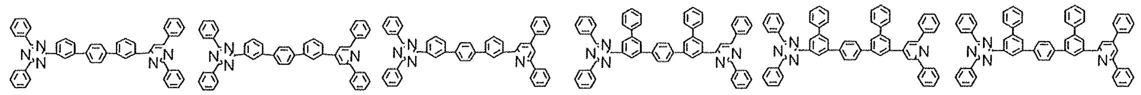
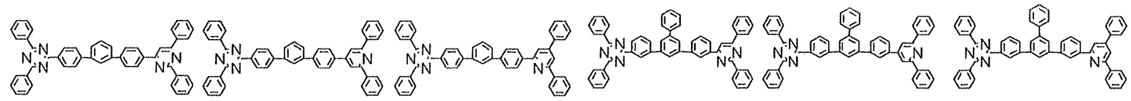
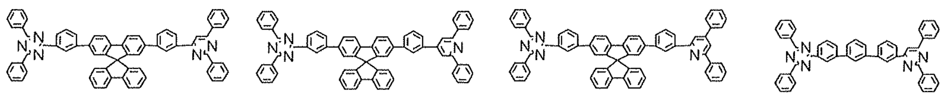
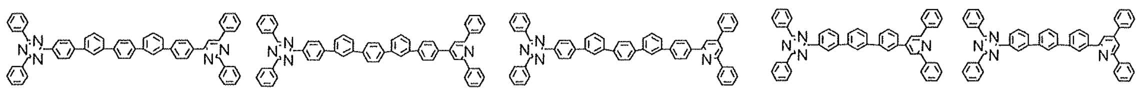
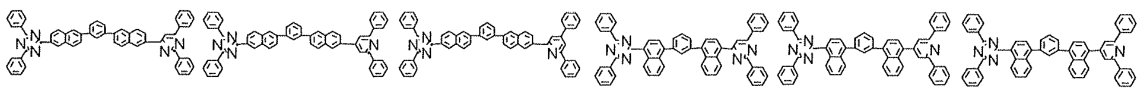
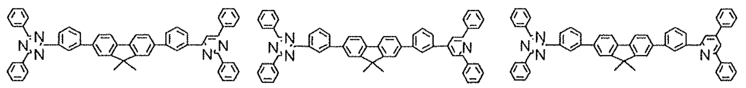
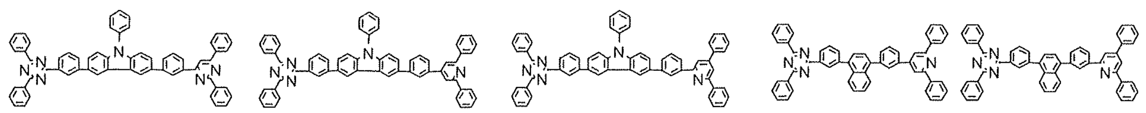
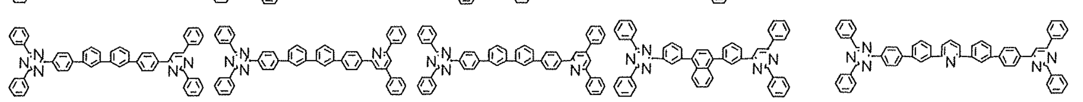
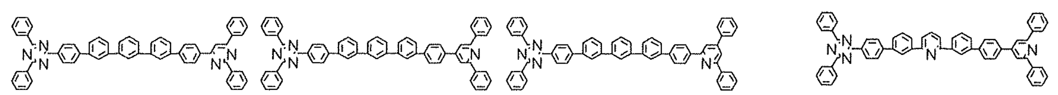

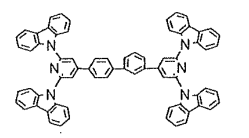
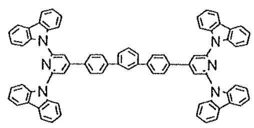
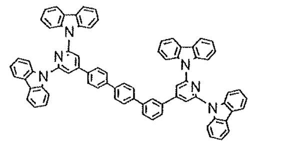
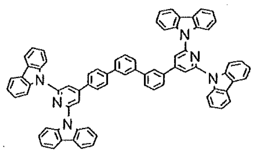
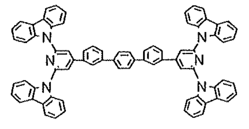
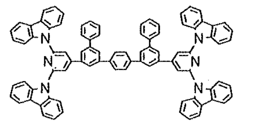
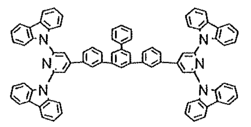
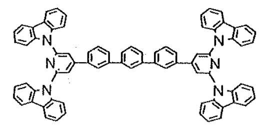
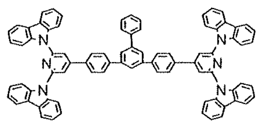
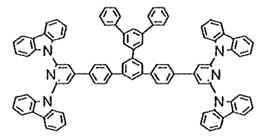
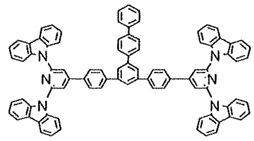
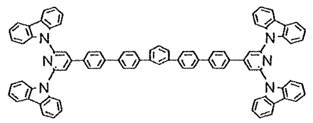
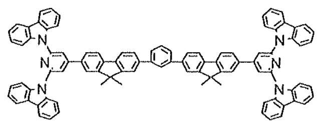
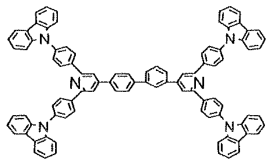
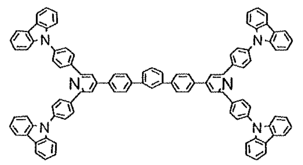
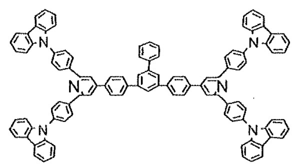
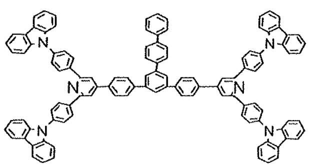
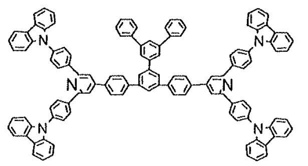
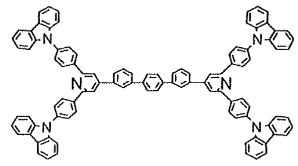
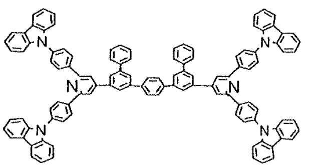
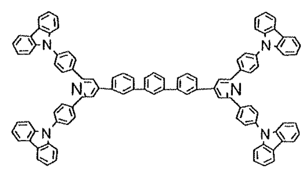
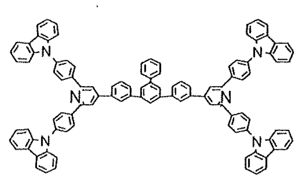
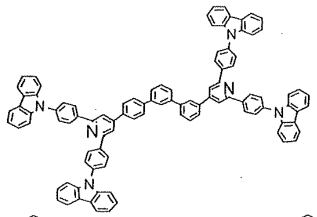
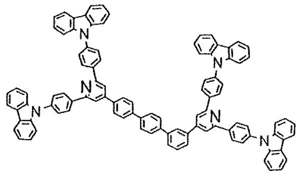
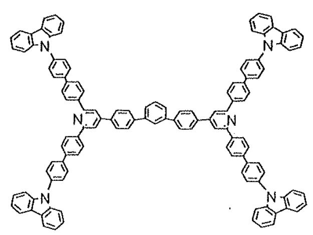
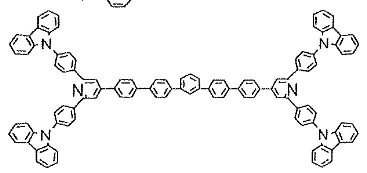
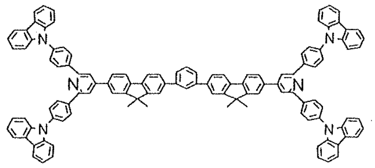

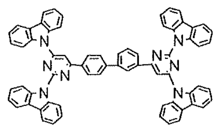 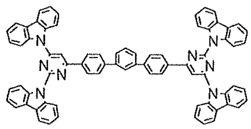 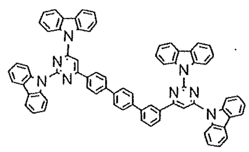 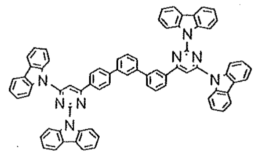

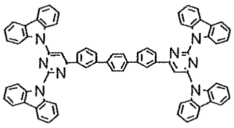 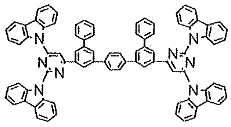 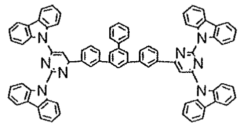 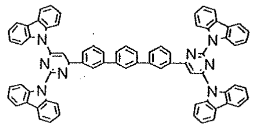

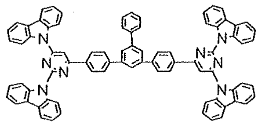 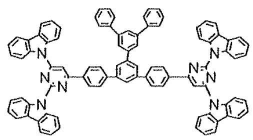 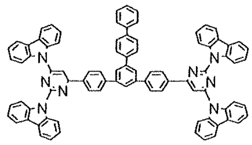

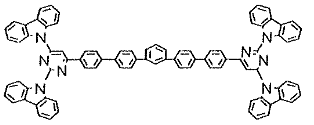 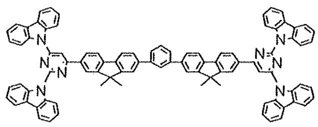

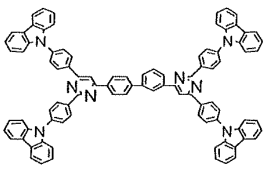 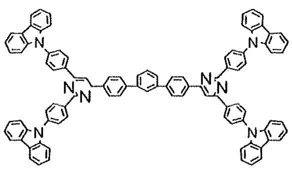 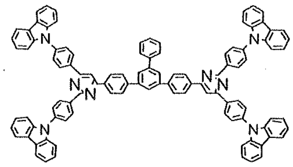

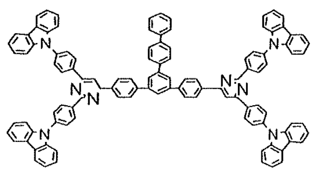 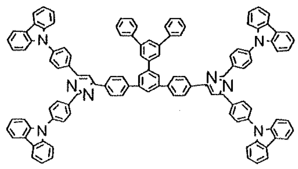 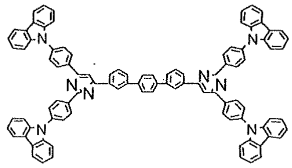

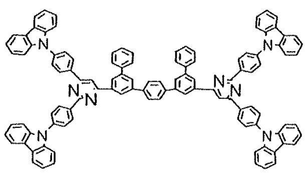 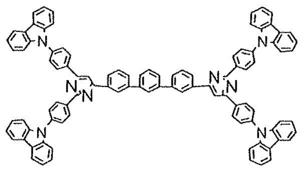 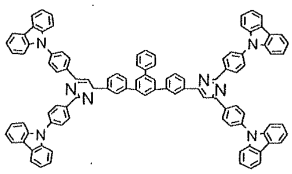

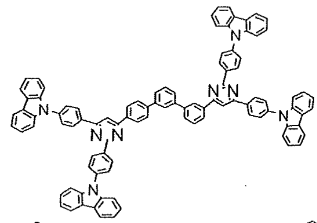 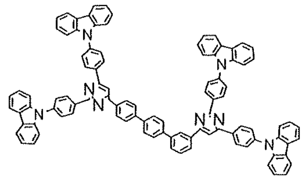 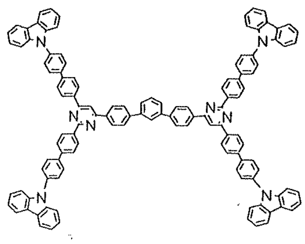

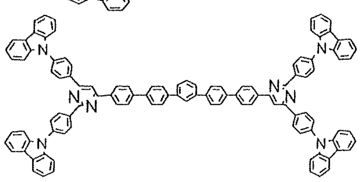 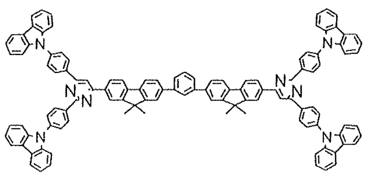

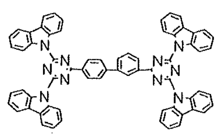
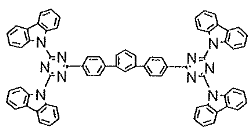
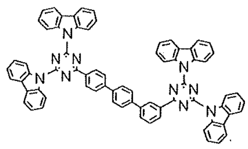
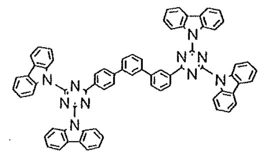
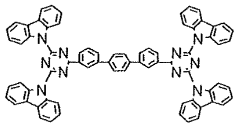
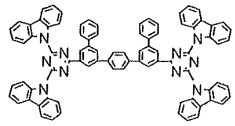
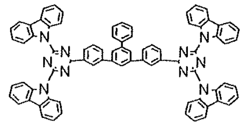
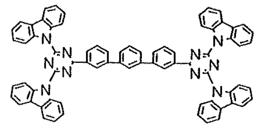
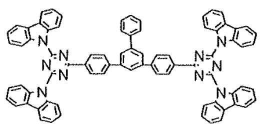
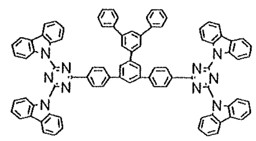
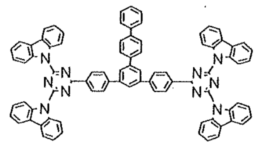
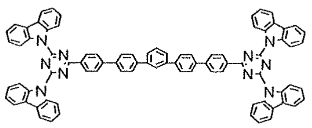
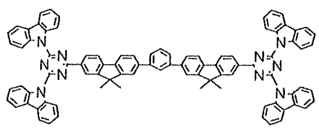
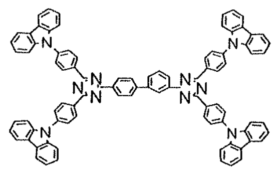
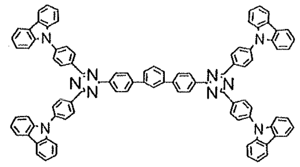
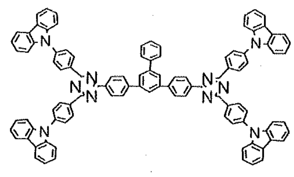
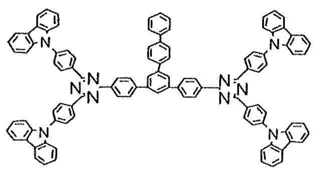
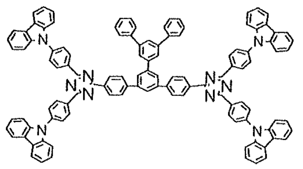
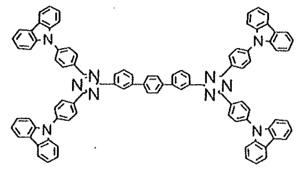
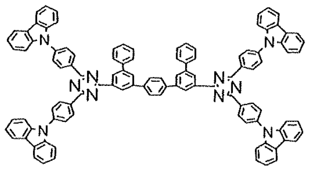
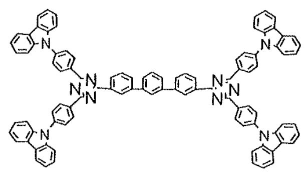
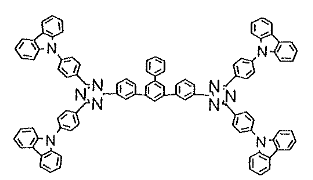
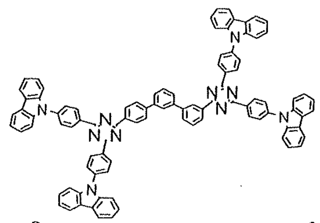
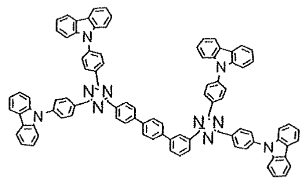
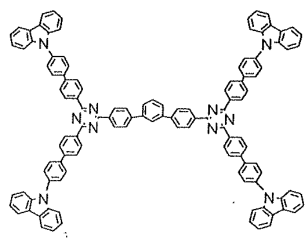
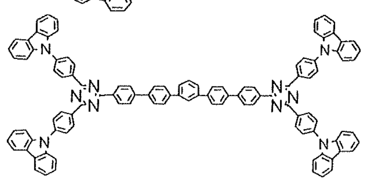
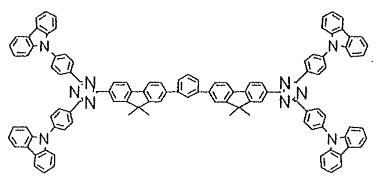

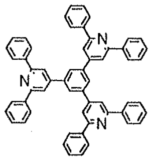
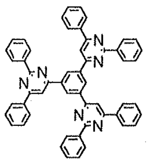
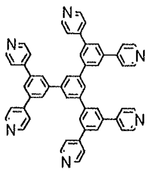
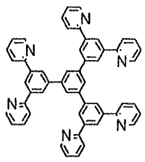

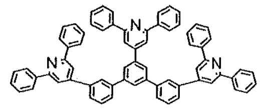
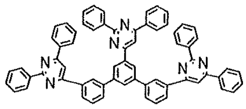
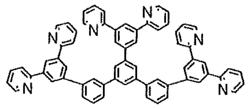

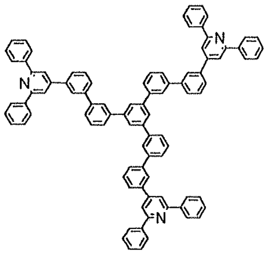
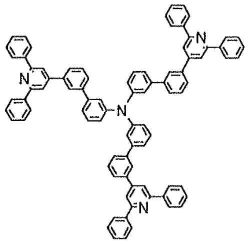
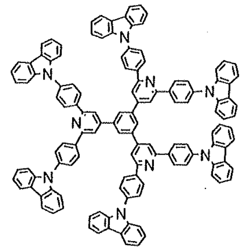

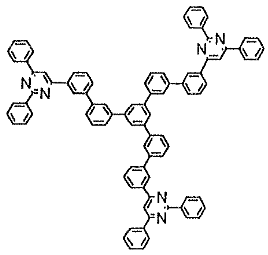
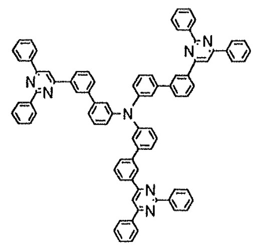
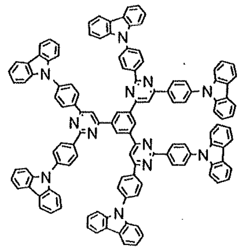

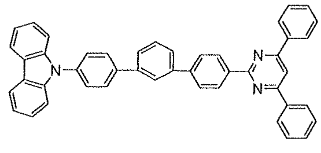
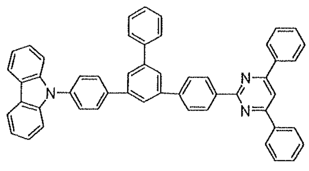
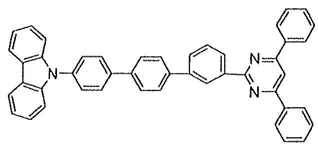

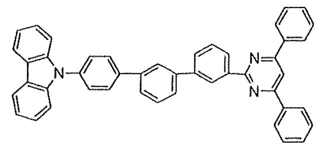
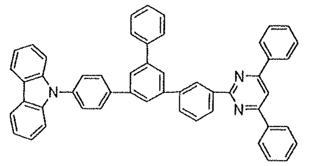
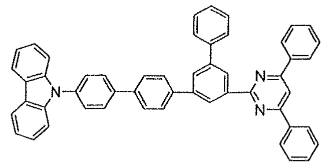

The material for an organic EL device composed of the compound represented by the general formula (1) of the present invention is preferably a host material in a light emitting layer of an organic electroluminescence device. The reason for this is as follows: when the host material is the compound represented by the general formula (1), a combination with a phosphorescent dopant to be described later enables the triplet exciton state of the compound represented by the general formula (1) to be effectively utilized even under a room temperature condition (20°C) . That is, the reason for the foregoing is that a fluorescent emission phenomenon can be caused by effectively transferring energy from a triplet state generated in the compound represented by the general formula (1) to the phosphorescent dopant.

In addition, the compound represented by the general formula (1) of the present invention has a glass transition temperature of preferably 120°C or higher, more preferably in the range of 120°C to 190°C, or still more preferably in the range of 140°C to 180°C. When the glass transition temperature is 120°C or higher, crystallization hardly occurs upon combination with the phosphorescent dopant, a long lifetime

is maintained, a short circuit hardly occurs in the case of passing electric current under a high-temperature environmental condition, and the use environment of the organic EL device is not limited. In addition, when the glass transition temperature is 190°C or lower, thermal decomposition hardly occurs upon film formation by means of vapor deposition, so the device can be easily handled. It should be noted that the glass transition temperature (Tg) can be determined to be the point at which a specific heat changes obtained in a case of heating under, for example, a temperature increase condition of 10°C/min in a nitrogen circulation state by using a scanning calorimeter (DSC, differential scanning calorimetory).

An organic EL device of the present invention has an organic thin film layer composed of one or more layers including at least a light emitting layer, the organic thin film layer being interposed between a cathode and an anode. In the organic EL device, at least one layer of the organic thin film layer contains the material for an organic EL device of the present invention.
Typical examples of the device constitution of the organic EL device of the present invention include, but not limited to:

(1) anode/light emitting layer/cathode
(2) anode/hole injecting layer/light emitting layer/cathode
(3) anode/light emitting layer/electron injecting layer/cathode
(4) anode/hole injecting layer/light emitting layer/electron injecting layer/cathode
(5) anode/organic semiconductor layer/light emitting layer/cathode
(6) anode/organic semiconductor layer/electron barrier layer/light emitting layer/cathode
(7) anode/organic semiconductor layer/light emitting layer/adhesiveness improving layer/cathode
(8) anode/hole injecting layer/hole transporting layer/light emitting layer/electron injecting layer/cathode
(9) anode/insulating layer/light emitting layer/insulating layer/cathode
(10) anode/inorganic semiconductor layer/insulating layer/light emitting layer/insulating layer/cathode
(11) anode/organic semiconductor layer/insulating layer/light emitting layer/insulating layer/cathode
(12) anode/insulating layer/hole injecting layer/hole transporting layer/light emitting layer/insulating layer/cathode
(13) anode/insulating layer/hole injecting layer/hole transporting layer/light emitting layer/electron injecting layer/cathode

In the organic EL device of the present invention, the light emitting layer contains a host material and a phosphorescent material. It is preferable that the host material contain the material for an organic EL device of the present invention, and it is more preferable that the host material be composed of the material for an organic EL device of the present invention.
At this time, the triplet energy E1 of the compound represented by the general formula (1) in the light emitting layer and a value of the triplet energy E2 of the phosphorescent dopant in the layer preferably satisfy the relationship of E1 > E2. That is, a combination of the compound represented by the general formula (1) and the phosphorescent dopant in such triplet energy relationship enables the triplet exciton state of the compound to be reliably utilized even under a room temperature condition. That is, a fluorescent emission phenomenon can be caused by reliably transferring energy from a triplet state generated in the compound represented by the general formula (1) to the phosphorescent dopant.

The phosphorescent material (phosphorescent dopant) is preferably a metal complex containing at least one metal selected from the group consisting of Ir, Ru, Pd, Pt, Os, and Re. The reason for this is that energy can be effectively transferred from a triplet exciton of the compound represented by the general formula (1) when the phosphorescent material is a complex of any one of those metals.
Examples of the metal complex include metal complexes such as tris(2-phenylpyridine)iridium, tris(2-phenylpyridine) ruthenium, tris(2-phenylpyridine)palladium, bis(2-phenylpyridine)platinum, tris(2-phenylpyridine)osmium, tris(2-phenylpyridine)rhenium, platinum octaethyl porphyrin, platinum octaphenyl porphyrin, palladium octaethyl porphyrin, and palladium octaphenyl porphyrin. In order that energy may be transferred with improved effectiveness for fluorescent emission, a metal complex containing Ir such as tris (2-phenylpyridine) iridium represented by the following formula is more preferable.

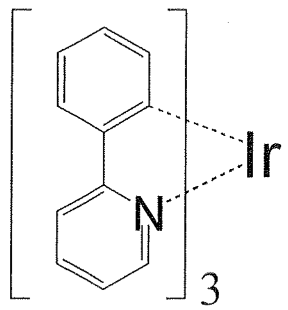

In addition, at least one ligand of the metal complex preferably has at least one skeleton selected from the group consisting of a phenylpyridine skeleton, a bipyridyl skeleton, and a phenanthroline skeleton. The reason for this is that the presence of any one of those electron-withdrawing skeletons in a molecule enables energy to be effectively transferred from a triplet exciton of the compound represented by the general formula (1). In particular, a material having a phenylpyridine skeleton among those skeletons such as tris(2-phenylpyridine)iridium is more preferable.
In the present invention, the loading of the phosphorescent material is preferably 0.1 to 30 parts by weight, more preferably 0.5 to 20 parts by weight, or still more preferably 1 to 15 parts by weight with respect to 100 parts by weight of the compound (host material) represented by the general formula (1). The reason for this is as follows: when the loading of the phosphorescent material is 0.1 part by weight or more, an effect of the addition of the material is exerted, and energy can be effectively transferred from a triplet exciton of the compound represented by the general formula (1) while when the loading is 30 parts by weight or less, the phosphorescent material can be uniformly blended with ease, and emission luminance does not vary.
A known method such as a deposition method, a spin coating method, or an LB method is an applicable method of forming the light emitting layer in the present invention.
In addition, according to the present invention, any other known light emitting material (such as PVK, PPV, CBP, Alq, BAlq, or a known complex) may be incorporated into the light emitting layer as desired to the extent that a material for the organic EL device of the present invention is not impaired.
The organic EL device of the present invention may be provided with a hole injecting layer having a thickness of 5 nm to 5 μm. Providing such hole injecting layer improves the injection of a hole into the light emitting layer, so high emission luminance can be obtained, or the device can be driven at a low voltage. In addition, a compound having a hole mobility measured when a voltage in the range of $1 \times 10^4$ to $1 \times 10^6$ V/cm is applied of $1 \times 10^{-6}$ $cm^2/V \cdot sec$ or more and an ionization energy of 5.5 eV or less is preferably used in the hole injecting layer. Examples of such material for the hole injecting layer include a porphyrin compound, an aromatic tertiary amine compound, a styrylamine compound, an aromatic dimethylidine-based compound, and a condensed aromatic ring compound. More specific examples of the material include organic compounds such as 4,4'-bis [N-(1-naphthyl)-N-phenylamino]biphenyl (hereinafter, abbreviated as "NPD") and 4,4',4"-tris[N-(3-methylphenyl)-N-phenylamino]triphenylamine (hereinafter, abbreviated as "MTDATA").
It is also more preferable to laminate two or more hole injecting layers as required. In this case, if the anode, a hole injecting layer 1 (hole injecting material 1), a hole injecting layer 2 (hole injecting material 2), · · · ·, and the light emitting layer are laminated in the stated order, the ionization energies (Ip) of the hole injecting materials preferably satisfy the relationship of Ip (hole injecting material 1) < Ip (hole injecting material 2) ... for reducing the driving voltage of the device.
In addition, it is also preferable to use an inorganic compound such as p-type Si or p-type SiC as a constituting component for the hole injecting layer. Further, it is also preferable to provide an organic semiconductor layer having a conductivity of $1 \times 10^{-10}$ s/cm or more for a gap between the hole injecting layer and the anode layer or between the hole injecting layer and the light emitting layer. Providing such organic semiconductor layer additionally improves the injection of a hole into the light emitting layer.
The organic EL device of the present invention may be provided with an electron injecting layer having a thickness of 5 nm to 5 μm. Providing such electron injecting layer improves the injection of an electron into the light emitting layer, so high emission luminance can be obtained, or the device can be driven at a low voltage. In addition, a compound having an electron mobility measured when a voltage in the range of $1 \times 10^4$ to $1 \times 10^6$ V/cm is applied of $1 \times 10^{-6}$ $cm^2/V \cdot sec$ or more and an ionization energy in excess of 5.5 eV is preferably used in the electron injecting layer. Examples of such material for the electron injecting layer include: a metal complex (A1 chelate: Alq) of 8-hydroxyquinoline or a derivative of the complex; and an oxadiazole derivative.
In addition, incorporating an alkali metal into the electron injecting layer can not only significantly reduce the voltage but also lengthen the lifetime.

The organic EL device of the present invention may be provided with a hole blocking layer having a thickness of 5 nm to 5 μm between the light emitting layer and the cathode. Providing such hole blocking layer improves the property with which a hole is confined in the organic light emitting layer, so high emission luminance can be obtained, or the device can be driven at a low voltage. Examples of such material for the hole blocking layer include 2,9-dimethyl-4,7-diphenyl-1,10-phenanthroline and 2,9-diethyl-4,7-diphenyl-1,10-phenanthroline. The hole blocking layer preferably further contains an alkali metal such as Li or Cs. As described above, a combination of a material for the hole blocking layer with an alkali metal can not only significantly reduce the voltage at which the organic EL device is driven but also lengthen the lifetime of the device. When an alkali metal is incorporated, the content of the alkali metal is preferably 0.01 to 30 wt%, more preferably 0.05 to 20 wt%, or still more preferably 0.1 to 15 wt% when the total amount of the hole blocking layer is defined as 100 wt%. The reason for this is as follows: when the content of the alkali metal is 0.01 wt% or more, an effect of the addition of the alkali metal is exerted while when the content is 30 wt% or less, the dispersibility of the alkali metal is so uniform that emission luminance does not vary.
In the present invention, a known method such as a deposition method, a spin coating method, or an LB method is an applicable method of forming the hole injecting layer, the electron injecting layer, or the hole blocking layer.
A reductive dopant is preferably added to an interfacial region between a cathode and an organic thin layer in the organic EL device of the present invention.
Examples of the reductive dopant include at least one kind selected from an alkali metal, an alkali metal complex, an alkali metal compound, an alkaline earth metal, an alkaline earth metal complex, an alkaline earth metal compound, a rare earth metal, a rare earth metal complex, a rare earth metal compound, and a halogen compound and an oxide thereof.
Examples of the alkali metal include Li having a work function of 2.93 eV, Na having a work function of 2.36 eV, K having a work function of 2.28 eV, Rb having a work function of 2.16 eV, and Cs having a work function of 1.95 eV, and an alkali metal having a work function of 3.0 eV or less is particularly preferable. Of those, Li, K, Rb, and Cs are preferable.
Examples of the alkali earth metal include Ca having a work function of 2.9 eV, Sr having a work function of 2.0 to 2.5 eV, and Ba having a work function of 2.52, eV, and an alkali earth metal having a work function of 3.0 eV or less is particularly preferable.
Examples of the rare earth metal include Sc, Y, Ce, Tb, and Yb, and a rare earth metal having a work function of 3.0 eV or less is particularly preferable.
Of those metals, a preferable metal has a particularly high reductive ability, so improvement of light emission intensity and long life of organic EL device can be attained by adding a relatively small amount of the metal to an electron injecting region.
Examples of the alkali metal compound include an alkali oxide such as $Li_2O$, $Cs_2O$, or $K_2O$, and an alkali halide such as LiF, NaF, CsF, or KF. Of those, an alkali oxide or an alkali fluoride such as LiF, $Li_2O$, or NaF is preferable.
Examples of the alkali earth metal compound include Bao, SrO, CaO, and mixtures thereof such as $Ba_xSr_{1-x}O$ ($0<x<1$) and $Ba_xCa_{1-x}O$ ($0<x<1$). Of those, BaO, Sro, and CaO are preferable.
Examples of the rare earth metal compound include $YbF_3$, $ScF_3$, $ScO_3$, $Y_2O_3$, $Ce_2O_3$, $GdF_3$, and $TbF_3$. Of those, $YbF_3$, $ScF_3$, and $TbF_3$ are preferable.
The alkali metal complex, alkali earth metal complex, and rare metal complex are not particularly limited as long as they each include as a metal ion at least one of alkali metal ions, alkali earth metal ions, and rare earth metal ions. Meanwhile, preferable examples of a ligand include, but not limited to, quinolinol, benzoquinolinol, acridinol, phenanthridinol, hydroxyphenyloxazole, hydroxyphenylthiazole, hydroxydiaryloxadiazole, hydroxydiarylthiadiazole, hydroxyphenylpyridine, hydroxyphenylbenzoimidazole, hydroxybenzotriazole, hydroxyfluborane, bipyridyl, phenanthroline, phthalocyanine, porphyrin, cyclopentadiene, β-dilcetones, azomethines, and derivatives thereof.
For the addition form of the reductive dopant, it is preferable that the reductive dopant be formed in a shape of a layer or an island in the interfacial region. A preferable example of the forming method includes a method in which an organic substance which is a light emitting material or an electron injecting material for forming the interfacial region is deposited at the same time as the reductive dopant is deposited by a resistant heating deposition method, thereby dispersing the reductive dopant in the organic substance. The disperse concentration by molar ratio of the organic compound to the reductive dopant is 100: 1 to 1:100, and is preferably 5:1 to 1:5.
In a case where the reductive dopant is formed into the shape of a layer, the light emittingmaterial or electron injecting material which serves as an organic layer in the interface is formed into the shape of a layer. After that, the reductive dopant is solely deposited by the resistant heating deposition method to form a layer preferably having a thickness of 0.1 to 15 nm.
In a case where the reductive dopant is formed into the shape of an island, the light emitting material or electron injecting material which serves as an organic layer in the interface is formed into the shape of an island. After that, the reductive dopant is solely deposited by the resistant heating deposition method to form an island preferably having a thickness of 0.05 to 1 nm.
The anode in the organic EL device of the present invention corresponds to a lower electrode or to a counter electrode depending on the constitution of an organic EL display device. A metal, alloy, or electroconductive compound

having a large work function (for example, 4.0 eV or more), or a mixture of them is preferably used in the anode. To be specific, each of electrode materials such as an indium tin oxide (ITO), an indium zinc oxide (IZO), copper iodide (CuI), tin oxide ($SnO_2$), zinc oxide (ZnO), gold, platinum, and palladium is preferably used alone, or two or more kinds of those electrode materials are preferably used in combination. The use of any one of those electrode materials enables an anode having a uniform thickness to be formed by employing a method with which a film can be formed in a dry state such as a vacuum deposition method, a sputtering method, an ion plating method, an electron beam deposition method, a CVD (chemical vapor deposition) method, a MOCVD (metal oxide chemical vapor deposition) method, or a plasma CVD method. When electroluminescence is extracted from the anode, the anode must be a transparent electrode. In that case, a conductive transparent material such as ITO, IZO, CuI, $SnO_2$, or ZnO is preferably used so that a value for a transmittance for electroluminescence is 70% or more. In addition, the thickness of the anode is not particularly limited; provided that a value for the thickness is preferably in the range of 10 to 1,000 nm, or more preferably 10 to 200 nm. The reason for this is as follows: when the thickness of the anode has a value in such range, a uniform thickness distribution and a transmittance for electroluminescence of 70% or more can be obtained while a value for the sheet resistance of the anode can be 1,000 Ω/□ or less, or more preferably 100 Ω/□ or less. It is also preferable to cause an arbitrary pixel in a light emitting surface to emit light by: sequentially providing the anode (lower electrode), an organic light emitting medium, and the cathode (counter electrode); and constituting the lower electrode and the counter electrode in an XY matrix fashion. That is, constituting the anode or the like as described above enables various pieces of information to be easily displayed in the organic EL device.

The cathode in the organic EL device of the present invention also corresponds to a lower electrode or to a counter electrode depending on the constitution of the organic EL device. A metal, alloy, or electroconductive compound having a small work function (for example, less than 4.0 eV), or a mixture of them or a product containing them is preferably used. To be specific, each of electrode materials each composed of, for example, any one of: a metal selected from sodium, a sodium-potassium alloy, cesium, magnesium, lithium, a magnesium-silver alloy, aluminum, aluminum oxide, an aluminum-lithium alloy, indium, and a rare earth metal; a mixture of any one of those metals and a material for the organic thin film layer; and a mixture of any one of these metals and a material for the electron injecting layer is preferably used alone, or two or more kinds of these electrode materials are preferably used in combination. In addition, as in the case of the anode, the thickness of the cathode is not particularly limited; provided that, to be specific, a value for the thickness is preferably in the range of 10 to 1,000 nm, or more preferably 10 to 200 nm. Further, when electroluminescence is extracted from the cathode, the cathode must be a transparent electrode. In that case, a value for a transmittance for electroluminescence is preferably 70% or more. As in the case of the anode, the cathode is preferably formed by employing a method with which a film can be formed in a dry state such as a vacuum deposition method or a sputtering method.

A support substrate in the organic EL device of the present invention is preferably excellent in mechanical strength and preferably has small permeability of moisture or of oxygen. Specific examples of the support substrate include a glass sheet, a metal sheet, a ceramic sheet, and a plastic sheet (made of, for example, a polycarbonate resin, an acrylic resin, a vinyl chloride resin, a polyethylene terephthalate resin, a polyimide resin, a polyester resin, an epoxy resin, a phenol resin, a silicon resin, or a fluorine resin). In addition, a support substrate composed of any one of those materials preferably further has an inorganic film formed on the support substrate, or is preferably subjected to a moisture-resistant treatment or hydrophobic treatment as a result of the application of a fluorine resin in order that moisture may be prevented from entering the organic EL device. In addition, a moisture content and a gas permeability coefficient in the support substrate are preferably small in order that moisture may be prevented from entering, in particular, the organic thin film layer. To be specific, the moisture content and gas permeability coefficient of the support substrate are preferably 0.0001 wt% or less and $1 \times 10^{-13}$ cc·cm/cm$^2$·sec·cmHg or less, respectively.

## EXAMPLES

Next, the present invention will be described in more detail by way of examples.

Synthesis Example 1 (Synthesis of Compound (H-1))

Compound (H-1) was synthesized as described below.

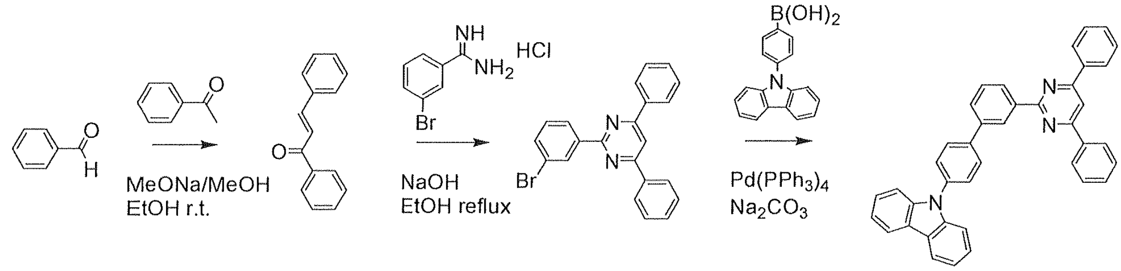


(Intermediate a) (H-1)

10.6 g (100 mmol) of benzaldehyde and 12.0 g (100 mmol) of acetophenone were loaded into a 300-ml three-necked flask, followed by argon replacement. Next, 200 ml of ethanol and 10 ml of a 1N solution of sodium methoxide in methanol were added, and the whole was stirred at room temperature for 5 hours. After that, the temperature of the resultant was increased in an oil bath at 70°C, and the resultant was reacted for an additional 4 hours while ethanol was refluxed. Next, 14.1 g (60 mmol) of 3-bromobenzamidine hydrochloride and 8.00 g (200 mmol) of sodium hydroxide were added, the temperature of the whole was increased in an oil bath at 70°C, and the whole was reacted for 5 hours. After completion of the reaction, the precipitated product was separated by filtration and purified by means of silica gel column chromatography (developing solvent: methylene chloride), whereby 14.8 g of (Intermediate a) were obtained (38.3% yield).

2.71 g (7 mmol) of (Intermediate a), 2.41 g (8.4 mmol) of 4-(N-carbazolyl)phenylboric acid, and 0.291 g (0.25 mmol, 3% Pd) of tetrakis(triphenylphosphine)palladium(0) were loaded into a 100-ml three-necked flask, and the inside of the container was replaced with argon. Further, 26 ml of 1, 2-dimethoxyethane and 12. 5 ml (3 eq) of a 2M aqueous solution of sodium carbonate were added, and the whole was refluxed under heat in an oil bath at 90°C for 9 hours. Afterone night,ion-exchanged water and methylene chloride were added to extract an organic layer, and the layer was washed with ion-exchanged water and a saturated salt solution. The resultant was dried with anhydrous magnesium sulfate, and the solvent was removed by distillation. 3.65 g of a gray solid as a residue were purified by means of silica gel column chromatography (developing solvent: hexane/methylene chloride), and the solvent was removed by distillation, whereby 3.15 g of Compound (H-1) were obtained. The result of the measurement of the FD-MS (field desorption mass spectrum) of obtained Compound (H-1) is shown below. FD-MS: calcd for $C_{40}H_{27}N_3$ = 550, found m/z = 550 ($M^+$, 100)

Synthesis Example 2 (Synthesis of Compound (H-2))

Compound (H-2) was synthesized as described below.

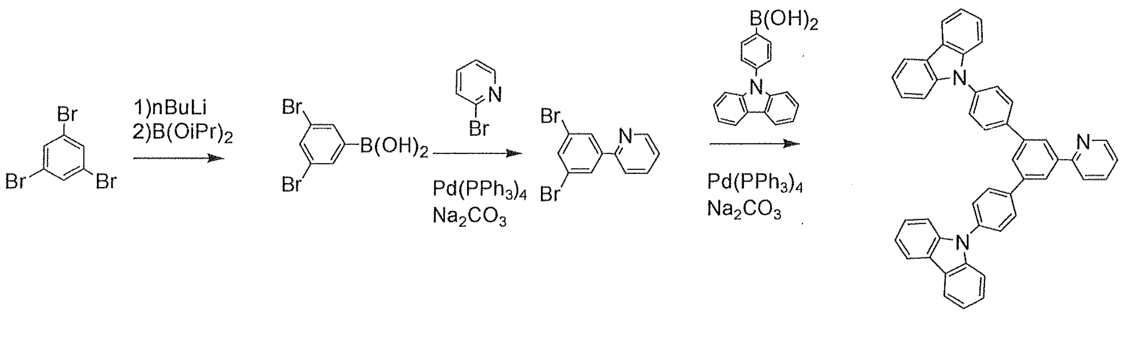


(Intermediate b) (Intermediate c) (H-2)

3.15 g (10 mmol) of tribromobenzene were dissolved in 40 ml of dehydrated ether. 7.8 ml (12.5 mmol) of a 1.6N solution of n-butyllithium in hexane were added to the solution under an argon atmosphere at -40°C, and the whole

was subjected to a reaction at -40°C to 0°C for 1 hour. Next, the temperature of the resultant was cooled to -70°C, 4.8 ml (21 mmol) of triisopropyl borate were dropped to the resultant, and the whole was stirred at -70°C for 1 hour. After that, the temperature of the resultant was increased to room temperature, and the resultant was subjected to a reaction for 6 hours. Further, 40 ml of 5% hydrochloric acid were dropped to the reaction solution, and then the whole was stirred at room temperature for 45 minutes. After the reaction solution had been separated into two layers, an organic layer was washed with a saturated salt solution and dried with anhydrous sodium sulfate. After the organic solvent had been removed by distillation under reduced pressure to about one fifth, the precipitated crystal was filtered, and was sequentially washed with a mixed solvent of toluene and normal-hexane, and normal-hexane, whereby 2.0 g of (Intermediate b) were obtained (7 mmol, 70% yield).

2.0 g (7 mmol) of (Intermediate b), 1.3 g (8.4 mmol) of 2-bromopyridine, 2.41 g (8.4 mmol) of 4-(N-carbazolyl)phenylboric acid, and 0.291 g (0.25 mmol, 3% Pd) of tetrakis(triphenylphosphine)palladium(0) were loaded into a 100-ml three-necked flask, and the inside of the container was replaced with argon. Further, 26 ml of 1,2-dimethoxyethane and 12.5 ml (3 eq) of a 2M aqueous solution of sodium carbonate were added, and the whole was refluxed under heat in an oil bath at 90°C for 9 hours. After one night, ion-exchanged water and methylene chloride were added to extract an organic layer, and the layer was washed with ion-exchanged water and a saturated salt solution. The resultant was dried with anhydrous magnesium sulfate, and the solvent was removed by distillation. 4.11 g of a gray solid as a residue were purified by means of silica gel column chromatography (developing solvent: hexane/methylene chloride), whereby 1.8 g of Intermediate (c) were obtained (5.75 mmol, 82% yeild).

1.8 g (5.75 mmol) of (Intermediate c), 1.98 g (6.9 mmol) of 4-(N-carbazolyl)phenylboric acid, and 0.239 g (0.21 mmol, 3% Pd) of tetrakis(triphenylphosphine)palladium(0) were loaded into a 100-ml three-necked flask, and the inside of the container was replaced with argon. Further, 20 ml of 1, 2-dimethoxyethane and 10. 5 ml (3 eq) of a 2M aqueous solution of sodium carbonate were added, and the whole was refluxed under heat in an oil bath at 90°C for 9 hours. After one night, ion-exchanged water and methylene chloride were added to extract an organic layer, and the layer was washed with ion-exchanged water and a saturated salt solution. The resultant was dried with anhydrous magnesium sulfate, and the solvent was removed by distillation. 2.57 g of a gray solid as a residue were purified by means of silica gel column chromatography (developing solvent: hexane/methylene chloride), and the solvent was removed by distillation, whereby 2.32 g of Compound (H-2) were obtained. The result of the measurement of the FD-MS of obtained Compound (H-2) is shown below.

FD-MS: calcd for $C_{47}H_{31}N_3$ = 638, found m/z = 638 ($M^+$, 100)

Synthesis Example 3 (Synthesis of Compound (H-3))

Compound (H-3) was synthesized as described below.

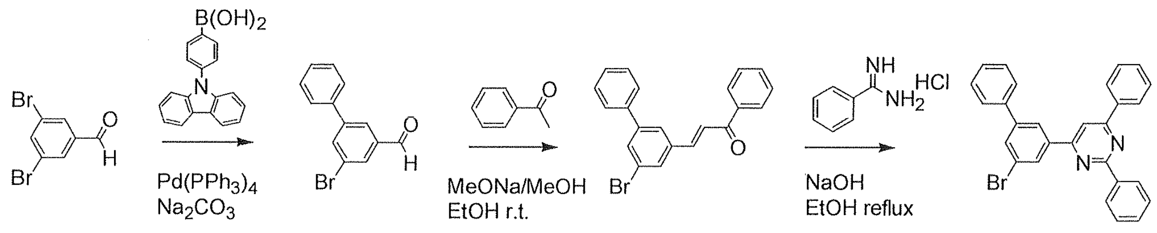


(Intermediate c) (Intermediate d)

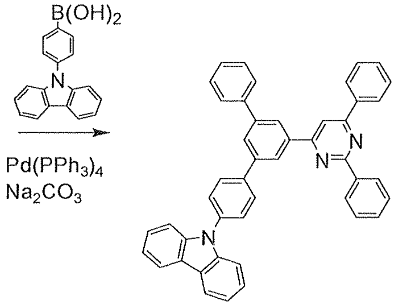


(H-3)

13.2g (50 mmol) of 3,5-dibromobenzaldehyde, 6.1 g (50 mmol) of phenylboric acid, and 1.73 g (1.5 mmol, 3% Pd) of tetrakis (triphenylphosphine) palladium (0) were loaded into a 300-ml three-necked flask, and the inside of the container was replaced with argon. Further, 100 ml of 1,2-dimethoxyethane and 75 ml (3 eq) of a 2M aqueous solution of sodium carbonate were added, and the whole was refluxed under heat in an oil bath at 90°C for 9 hours. After one night, ion-exchanged water and methylene chloride were added to extract an organic layer, and the layer was washed with ion-exchanged water and a saturated salt solution. The resultant was dried with anhydrous magnesium sulfate, and the solvent was removed by distillation. 10.3 g of a gray solid as a residue were purified by means of silica gel column chromatography (developing solvent: hexane/methylene chloride), whereby 9.1 g of (Intermediate c) were obtained (35 mmol, 70% yeild).

9.1 g (35 mmol) of (Intermediate c) and 4.2 g (35 mmol) of acetophenone were loaded into a 300-ml three-necked flask, followed by argon replacement. Next, 150 ml of ethanol and 7 ml of a 1N solution of sodium methoxide in methanol were added, and the whole was stirred at room temperature for 5 hours. After that, the temperature of the resultant was increased in an oil bath at 70°C, and the resultant was reacted for an additional 4 hours while ethanol was refluxed. Next, 6.58 g (42 mmol) of benzamidine hydrochloride and 5.6 g (140 mmol) of sodium hydroxide were added, the temperature of the whole was increased in an oil bath at 70°C, and the whole was reacted for 5 hours. After completion of the reaction, the precipitated product was separated by filtration and purified by means of silica gel column chromatography (developing solvent: methylene chloride), whereby 4. 6 g of (Intermediate d) were obtained (9.9 mmol, 28% yield) .

3.24 g (7 mmol) of (Intermediate d), 2.41 g (8.4 mmol) of 4-(N-carbazolyl)phenylboric acid, and 0.291 g (0.25 mmol, 3% Pd) of tetrakis(triphenylphosphine)palladium(0) were loaded into a 100-ml three-necked flask, and the inside of the container was replaced with argon. Further, 26 ml of 1, 2-dimethoxyethane and 12. 5 ml (3 eq) of a 2M aqueous solution of sodium carbonate were added, and the whole was refluxed under heat in an oil bath at 90°C for 9 hours. After one night, ion-exchanged water and methylene chloride were added to extract an organic layer, and the layer was washed with ion-exchanged water and a saturated salt solution. The resultant was dried with anhydrous magnesium sulfate, and the solvent was removed by distillation. 3.72 g of a gray solid as a residue were purified by means of silica gel column chromatography (developing solvent: hexane : methylene chloride = 8 : 2 ~ 5 : 5), and the solvent was removed by distillation, whereby 2.72 g of Compound (H-3) were obtained. The result of the measurement of the FD-MS of obtained Compound (H-3) is shown below.

FD-MS: calcd for $C_{46}H_{31}N_3$ = 626, found m/z = 626 ($M^+$, 100)

Synthesis Example 4 (Synthesis of Compound (H-4))

Compound (H-4) was synthesized as described below.

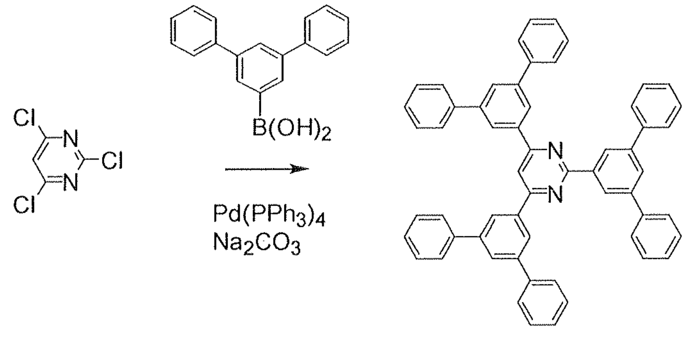


(H-4)

1.28 g (7 mmol) of 1,3,5-trichloropyrimidine, 6.91 g (25.2 mmol) of 3, 5-diphenylpheylboronic acid, and 0.58 g (0.50 mmol, 2% Pd) of tetrakis (triphenylphosphine) palladium (0) were loaded into a 100-ml three-necked flask, and the inside of the container was replaced with argon. Further, 40 ml of 1, 2-dimethoxyethane and 37.8 ml (3 eq) of a 2M aqueous solution of sodium carbonate were added, and the whole was refluxed under heat in an oil bath at 90°C for 9 hours. After one night, ion-exchanged water and methylene chloride were added to extract an organic layer, and the layer was washed with ion-exchanged water and a saturated salt solution. The resultant was dried with anhydrous magnesium sulfate, and the solvent was removed by distillation. 3.20 g of a gray solid as a residue were purified by means of silica gel column chromatography (developing solvent: hexane/methylene chloride), whereby 3.21 g of Compound (H-4) were obtained. The result of the measurement of the FD-MS of obtained Compound (H-4) is shown below.
FD-MS: calcd for $C_{58}H_{40}N_2$ = 765, found m/z = 765 ($M^+$, 100)

Synthesis Example 5 (Synthesis of Compound (H-5))

Compound (H-5) was synthesized as described below.

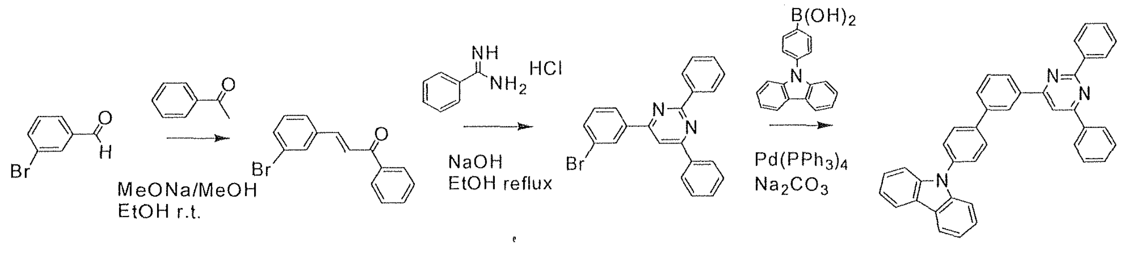


(Intermediate e) (H-5)

18.5 g (100 mmol) of 3-bromobenzaldehyde and 12.0 g (100 mmol) of acetophenone were loaded into a 300-ml three-necked flask, followed by argon replacement. Next, 200 ml of ethanol and 10 ml of a 1N solution of sodium methoxide in methanol were added, and the whole was stirred at room temperature for 5 hours. After that, the temperature of the resultant was increased in an oil bath at 70°C, and the resultant was reacted for an additional 4 hours while ethanol was refluxed. Next, 9.40 g (60 mmol) of benzamidine hydrochloride and 8.00 g (200 mmol) of sodium hydroxide were added, the temperature of the whole was increased in an oil bath at 70°C, and the whole was reacted for 5 hours. After the completion of the reaction, the precipitated product was separated by filtration and purified by means of silica gel column chromatography (developing solvent: methylene chloride), whereby 7.26 g of (Intermediate e) were obtained (25.3% yield).
2.71 g (7 mmol) of (Intermediate e), 2.41 g (8.4 mmol) of 4-(N-carbazolyl)phenylboric acid, and 0.291 g (0.25 mmol, 3% Pd) of tetrakis(triphenylphosphine)palladium(0) were loaded into a 100-ml three-necked flask, and the inside of the

container was replaced with argon. Further, 26 ml of 1, 2-dimethoxyethane and 12 . 5 ml (3 eq) of a 2M aqueous solution of sodium carbonate were added, and the whole was refluxed under heat in an oil bath at 90°C for 9 hours. After one night,ion-exchanged waterand methylene chloride were added to extract an organic layer, and the layer was washed with ion-exchanged water and a saturated salt solution. The resultant was dried with anhydrous magnesium sulfate, and the solvent was removed by distillation. 4.11 g of a gray solid as a residue were purified by means of silica gel column chromatography (developing solvent: hexane/methylene chloride), and the solvent was removed by distillation, whereby 2.75 g of white powder of Compound (H-5) were obtained. The result of the measurement of the FD-MS of obtained Compound (H-5) is shown below.
FD-MS: calcd for $C_{40}H_{27}N_3$ = 545, found m/z = 545 ($M^+$, 100)

Synthesis Example 6 (Synthesis of Compound (H-6))

Compound (H-6) was synthesized as described below.

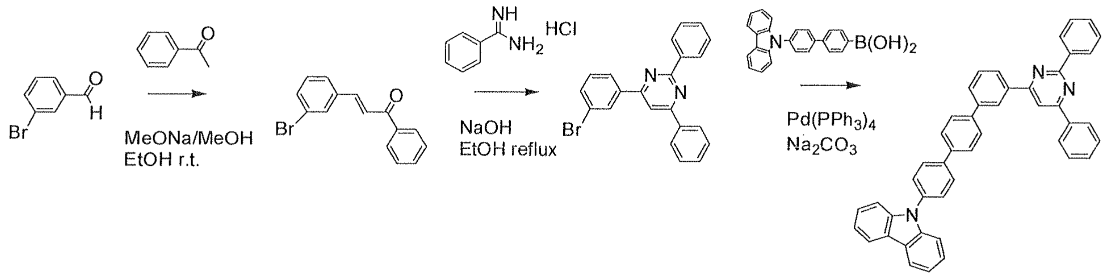


(H-6)

2.71 g (7 mmol) of (Intermediate e) synthesized in Synthesis Example 5, 3.05 g (8.4 mmol) of 4-(N-carbazolyl) biphenylboric acid, and 0.291 g (0.25 mmol, 3% Pd) of tetrakis(triphenylphosphine)palladium(0) were loaded into a 100-ml three-necked flask, and the inside of the container was replaced with argon. Further, 26 ml of 1, 2-dimethoxyethane and 12. 5 ml (3 eq) of a 2-M aqueous solution of sodium carbonate were added, and the whole was refluxed under heat in an oil bath at 90°C for 9 hours. After onenight,ion-exchanged waterand methylene chloride were added to extract an organic layer, and the layer was washed with ion-exchanged water and a saturated salt solution. The resultant was dried with anhydrous magnesium sulfate, and the solvent was removed by distillation. 3.97 g of a gray solid as a residue were purified by means of silica gel column chromatography (developing solvent: hexane/methylene chloride), and the solvent was removed by distillation, whereby 3.40 g of Compound (H-6) were obtained. The result of the measurement of the FD-MS of obtained Compound (H-6) is shown below.
FD-MS: calcd for $C_{46}H_{31}N_3$ = 626, found m/z = 626 ($M^+$, 100)

Synthesis Example 7 (Synthesis of Compound (H-7))

Compound (H-7) was synthesized as described below.

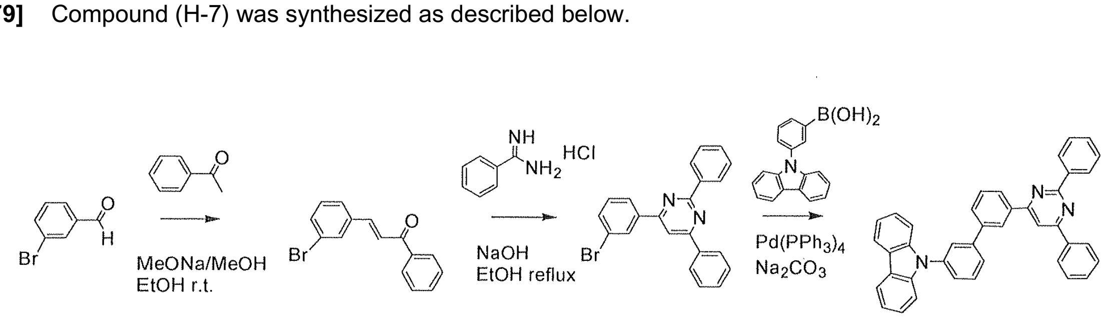


(H-7)

2.71 g (7 mmol) of (Intermediate e) synthesized in Synthesis Example 5, 2.41 g (8.4 mmol) of 3-(N-carbazolyl)phenylboric acid, and 0.291 g (0.25 mmol, 3% Pd) of tetrakis(triphenylphosphine)palladium(0) were loaded into a 100-ml three-necked flask, and the inside of the container was replaced with argon. Further, 26 ml of 1, 2-dimethoxyethane and 12. 5 ml (3 eq) of a 2-M aqueous solution of sodium carbonate were added, and the whole was refluxed under heat in an oil bath at 90°C for 9 hours . After one night, ion-exchanged water and methylene chloride were added to extract an organic layer, and the layer was washed with ion-exchanged water and a saturated salt solution. The resultant was dried with anhydrous magnesium sulfate, and the solvent was removed by distillation. 3.41 g of a gray solid as a residue were purified by means of silica gel column chromatography (developing solvent: hexane/methylene chloride), and the solvent was removed by distillation, whereby 2.64 g of Compound (H-7) were obtained. The result of the measurement of the FD-MS of obtained Compound (H-7) is shown below.
FD-MS: calcd for $C_{40}H_{27}N_3$ = 545, found m/z = 545 ($M^+$, 100)

Synthesis Example 8 (Synthesis of Compound (H-8))

Compound (H-8) was synthesized as described below.

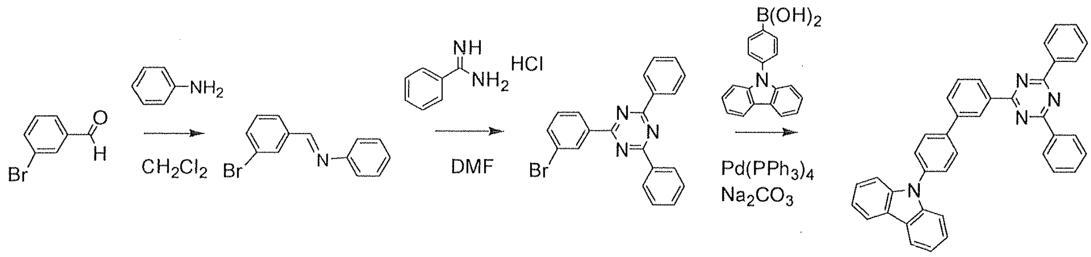


(Intermediate f) (Intermediate g) (H-8)

18.5 g (100 mmol) of 3-bromobezaldehyde and 9. 31 g (100 mmol) of aniline were loaded into a 300-ml three-necked flask, followed by argon replacement. Next, 200 ml of ethylene chloride and 10 ml of a 1N solution of sodium methoxide in methanol were added, and the whole was stirred at room temperature for 5 hours. Next, 9.40 g (60 mmol) of benzamidine hydrochloride and 8.00 g (200 mmol) of sodium hydroxide were added, the temperature of the whole was increased in an oil bath at 70°C, and the whole was reacted for 5 hours. After the completion of the reaction, the precipitated product was separated by filtration and purified by means of silica gel column chromatography (developing solvent: methylene chloride), whereby 12.8 g of (Intermediate f) were obtained (33.0% yield).
2.72 g (7 mmol) of (Intermediate g), 2.41 g (8.4 mmol) of 4-(N-carbazolyl-)phenylboric acid, and 0.291 g (0.25 mmol, 3% Pd) of tetrakis(triphenylphosphine)palladium(0) were loaded into a 100-ml three-necked flask, and the inside of the container was replaced with argon. Further, 26 ml of 1, 2-dimethoxyethane and 12. 5 ml (3 eq) of a 2M aqueous solution of sodium carbonate were added, and the whole was refluxed under heat in an oil bath at 90°C for 9 hours. After one night,ion-exchanged water and methylene chloride were added to extract an organic layer, and the layer was washed with ion-exchanged water and a saturated salt solution. The resultant was dried with anhydrous magnesium sulfate, and the solvent was removed by distillation. 3.88 g of a gray solid as a residue were purified by means of silica gel column chromatography (developing solvent: hexane/methylene chloride), and the solvent was removed by distillation, whereby 3.26 g of Compound (H-8) were obtained. The result of the measurement of the FD-MS of obtained Compound (H-8) is shown below.
FD-MS: calcd for $C_{39}H_{26}N_4$ = 551, found m/z = 551 ($M^+$, 100)

Synthesis Example 9 (Synthesis of Compound (H-9))

Compound (H-9) was synthesized as described below.

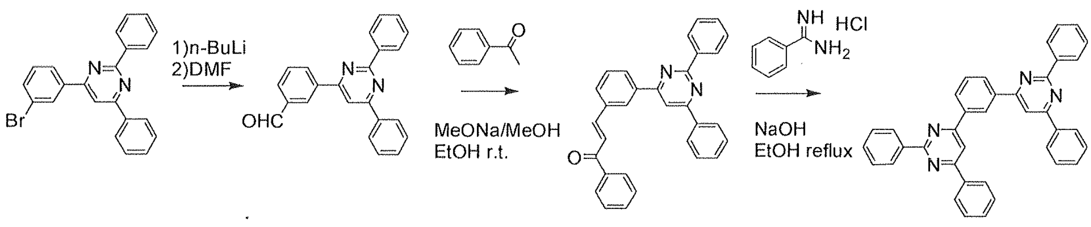


(Intermediate h) (H-9)

38.7 g (100 mmol) of (Intermediate e) synthesized in the same manner as in Synthesis Example 5 were dissolved in 300 ml of ether in a 1,000-ml three-necked flask. 75 ml (120 mmol) of a solution (1.6 M) of normal-butyllithium in hexane were added under an argon atmosphere at -16 to -42°C, and the whole was stirred at -42°C to 0°C for 1 hour. Next, the temperature of the reaction solution was cooled to -70 °C, 22 g (300 mmol) of dimethylformamide were dropped to the reaction solution, and the whole was stirred at -70°C for 1 hour. After that, the temperature of the resultant was increased to room temperature, and the resultant was stirred for 6 hours. Further, 200 ml of 5% hydrochloric acid were dropped to the reaction solution, and then the whole was stirred at room temperature for 45 minutes. After the reaction solution had been separated into two layers, an organic layer was sequentially washed with 3% hydrochloric acid and a saturated salt solution, and was dried with anhydrous sodium sulfate. After the organic solvent had been removed by distillation under reduced pressure to about one fifth, the precipitated crystal was filtered, and was sequentially washed with a mixed solvent of toluene and normal-hexane, and normal-hexane, whereby 23.5 g of (Intermediate h) were obtained (70% yield).
11.8 g (35 mmol) of (Intermediate h) and 4.20 g (35 mmol) of acetophenone were loaded into a 300-ml three-necked flask, followed by replacement with argon. Next, 80 ml of ethanol and 3.5 ml of a 1N solution of sodium methoxide in methanol were added, and the whole was stirred at room temperature for 5 hours. After that, the temperature of the resultant was increased in an oil bath at 70°C, and the resultant was subjected to a reaction for an additional 4 hours while ethanol was refluxed. Next, 3.28 g (21 mmol) of benzamidine hydrochloride and 2.80 g (70 mmol) of sodium hydroxide were added, the temperature of the whole was increased in an oil bath at 70 °C, and the whole was subjected to a reaction for 5 hours. After the completion of the reaction, the precipitated product was separated by filtration, and was purified by means of silica gel column chromatography (developing solvent: methylene chloride), whereby 4.71 g of Compound (H-9) were obtained. The result of the measurement of the FD-MS of obtained Compound (H-9) is shown below. FD-MS: calcd for $C_{38}H_{26}N_4$ = 539, found m/z = 551 ($M^+$, 100)

Synthesis Example 10 (Synthesis of Compound (H-10))

Compound (H-10) was synthesized as described below.

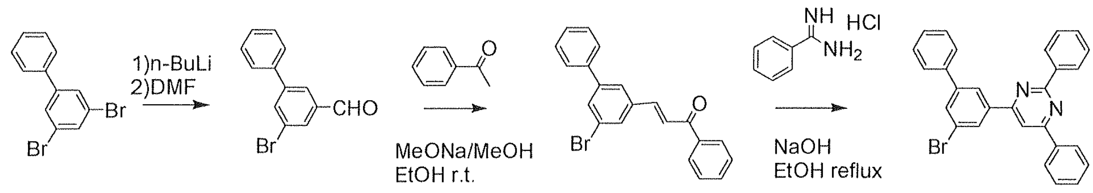


(Intermediate i) (Intermediate j)

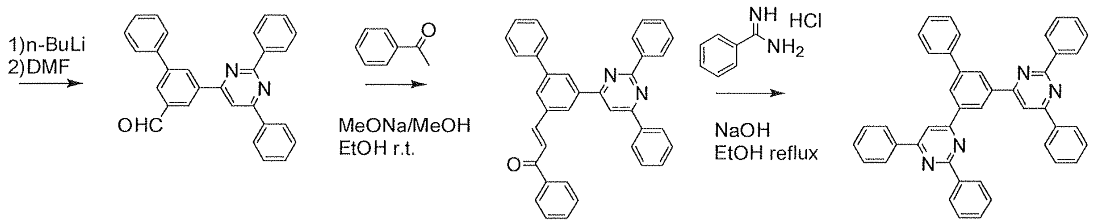


(Intermediate k) (H-10)

31.2 g (100 mmol) of 1-phenyl-3,5-dibromobenzene were dissolved in 300 ml of ether in a 1, 000-ml three-necked flask. 75 ml (120 mmol) of a solution (1.6M) of normal-butyllithium in hexane were added under an argon atmosphere at -16 to -42°C, and the whole was stirred at -42°C to 0°C for 1 hour. Next, the temperature of the reaction solution was cooled to -70 °C, 22 g (300 mmol) of dimethylformamide were dropped to the reaction solution, and the whole was stirred at - 70°C for 1 hour. After that, the temperature of the resultant was increased to room temperature, and the resultant was stirred for 6 hours. Further, 200 ml of 5% hydrochloric acid were dropped to the reaction solution, and then the whole was stirred at room temperature for 45 minutes. After the reaction solution had been separated into two layers, an organic layer was sequentially washed with 3% hydrochloric acid and a saturated salt solution, and was dried with anhydrous sodium sulfate. After the organic solvent had been removed by distillation under reduced pressure to about one fifth, the precipitated crystal was filtered, and was sequentially washed with a mixed solvent of toluene and normal-hexane, and normal-hexane, whereby 19.6 g of (Intermediate h) were obtained (75% yield).

18.3 g (70 mmol) of (Intermediate i) and 8.4 g (70 mmol) of acetophenone were loaded into a 300-ml three-necked flask, followed by replacement with argon. Next, 80 ml of ethanol and 7.0 ml of a 1N solution of sodium methoxide in methanol were added, and the whole was stirred at room temperature for 5 hours. After that, the temperature of the resultant was increased in an oil bath at 70°C, and the resultant was subjected to a reaction for an additional 4 hours while ethanol was refluxed. Next, 6.56 g (42 mmol) of benzamidine hydrochloride and 5.60 g (140 mmol) of sodium hydroxide were added, the temperature of the whole was increased in an oil bath at 70°C, and the whole was subjected to a reaction for 5 hours. After the completion of the reaction, the precipitated product was separated by filtration, and was purified by means of silica gel column chromatography (developing solvent: methylene chloride), whereby 13.9 g of (Intermediate j) were obtained (42.9% yield).

11.6 g (25 mmol) of (Intermediate j) were dissolved in 100 ml of ether in a 500-ml three-necked flask. 19 ml (30 mmol) of a solution (1.6 M) of normal-butyllithium in hexane were added under an argon atmosphere at -16 to -42°C, and the whole was stirred at -42°C to 0°C for 1 hour. Next, the temperature of the reaction solution was cooled to -70°C, 7.3g (100 mmol) of dimethylformamide were dropped to the reaction solution, and the whole was stirred at -70 °C for 1 hour. After that, the temperature of the resultant was increased to room temperature, and the resultant was stirred for 6 hours. Further, 200 ml of 5% hydrochloric acid were dropped to the reaction solution, and then the whole was stirred at room temperature for 45 minutes. After the reaction solution had been separated into two layers, an organic layer was sequentially washed with 3% hydrochloric acid and a saturated salt solution, and was dried with anhydrous sodium sulfate. After the organic solvent had been removed by distillation under reduced pressure to about one fifth, the precipitated crystal was filtered, and was sequentially washed with a mixed solvent of toluene and normal-hexane, and normal-hexane, whereby 8.0 g of (Intermediate k) were obtained (78% yield).

8.0 g (19.4 mmol) of (Intermediate k) and 2.33 g (19.4 mmol) of acetophenone were loaded into a 300-ml three-necked flask, followed by replacement with argon. Next, 30 ml of ethanol and 2.0 ml of a 1N solution of sodium methoxide in methanol were added, and the whole was stirred at room temperature for 5 hours. After that, the temperature of the resultant was increased in an oil bath at 70°C, and the resultant was subjected to a reaction for an additional 4 hours while ethanol was refluxed. Next, 1.81 g (11.6 mmol) of benzamidine hydrochloride and 1.55 g (38.8 mmol) of sodium hydroxide were added, the temperature of the whole was increased in an oil bath at 70°C, and the whole was subjected

to a reaction for 5 hours. After the completion of the reaction, the precipitated product was separated by filtration, and was purified by means of silica gel column chromatography (developing solvent: methylene chloride), whereby 4.61 g of Compound (H-10) were obtained. The result of the measurement of the FD-MS of obtained Compound (H-10) is shown below.
FD-MS: calcd for $C_{44}H_{30}N_4$ = 615, found m/z = 615 ($M^+$, 100)

Synthesis Example 11 (Synthesis of Compound (H-11))

Compound (H-11) was synthesized as described below.

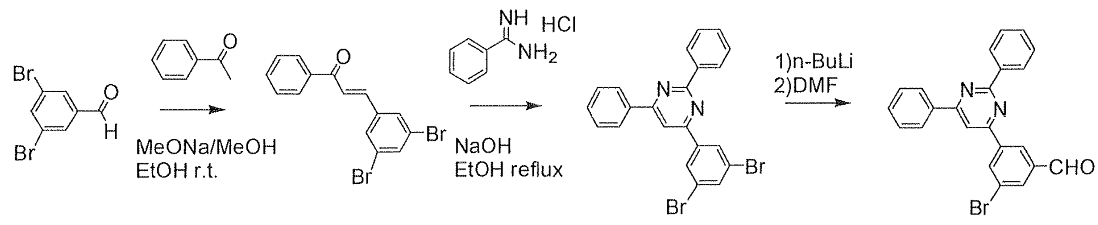


(Intermediate l) (Intermediate m)

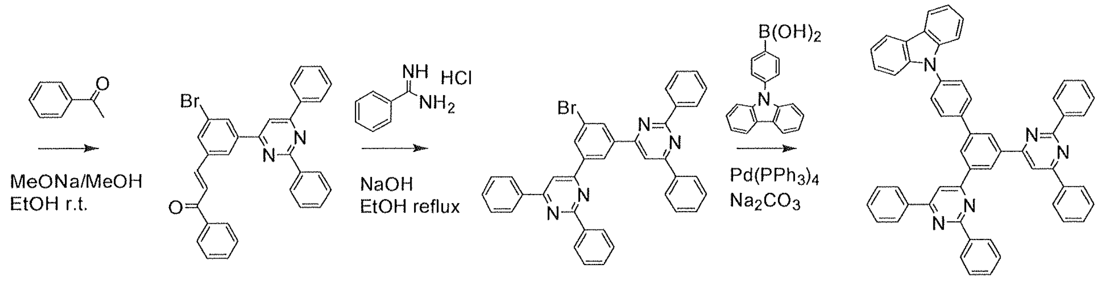


(Intermediate n) (H-11)

26.4 g (100 mmol) of 3,5-dibromobenzaldehyde and 12.0 g (10 mmol) of acetophenone were loaded into a 300-ml three-necked flask, followed by argon replacement. Next, 100 ml of ethanol and 10. 0 ml of a 1N solution of sodium methoxide in methanol were added, and the whole was stirred at room temperature for 5 hours. After that, the temperature of the resultant was increased in an oil bath at 70°C, and the resultant was reacted for an additional 4 hours while ethanol was refluxed. Next, 10.9 g (70 mmol) of benzamidine hydrochloride and 8 . 0 g (200 mmol) of sodium hydroxide were added, the temperature of the whole was increased in an oil bath at 70°C, and the whole was reacted for 5 hours. After the completion of the reaction, the precipitated product was separated by filtration and purified by means of silica gel column chromatography (developing solvent: methylene chloride), whereby 18.6 g of (Intermediate 1) were obtained (40% yield).
18.6 g (40 mmol) of (Intermediate 1) were dissolved in 150 ml of ether in a 1,000-ml three-necked flask. 40 ml (64 mmol) of a solution (1.6M) of normal-butyllithium in hexane were added under an argon atmosphere at -16 to -42°C, and the whole was stirred at -42°C to 0°C for 1 hour. Next, the temperature of the reaction solution was cooled to -70 °C, 8.8 g (120 mmol) of dimethylformamide were dropped to the reaction solution, and the whole was stirred at -70 °C for 1 hour. After that, the temperature of the resultant was increased to room temperature, and the resultant was stirred for 6 hours. Further, 100 ml of 5% hydrochloric acid were dropped to the reaction solution, and then the whole was stirred at room temperature for 45 minutes. After the reaction solution had been separated into two layers, an organic layer was sequentially washed with 3% hydrochloric acid and a saturated salt solution, and was dried with anhydrous sodium sulfate. After the organic solvent had been removed by distillation under reduced pressure to about one fifth, the precipitated crystal was filtered, and was sequentially washed with a mixed solvent of toluene and normal-hexane, and normal-hexane, whereby 13.8 g of (Intermediate m) were obtained (83% yield).

12.5 g (30 mmol) of (Intermediate m) and 3.6 g (30 mmol) of acetophenone were loaded into a 300-ml three-necked flask, followed by replacement with argon. Next, 70 ml of ethanol and 6.5 ml of a 1N solution of sodium methoxide in methanol were added, and the whole was stirred at room temperature for 5 hours. After that, the temperature of the resultant was increased in an oil bath at 70°C, and the resultant was subjected to a reaction for an additional 4 hours while ethanol was refluxed. Next, 3.28 g (21 mmol) of benzamidine hydrochloride and 2.40 g (60 mmol) of sodium hydroxide were added, the temperature of the whole was increased in an oil bath at 70°C, and the whole was subjected to a reaction for 5 hours. After the completion of the reaction, the precipitated product was separated by filtration, and was purified by means of silica gel column chromatography (developing solvent: methylene chloride), whereby 8.03 g of (Intermediate n) were obtained (43.3% yield).
4.32 g (7 mmol) of (Intermediate n), 2.41 g (8.4 mmol) of 4-(N-carbazolyl)phenylboric acid, and 0.291 g (0.25 mmol, 3% Pd) of tetrakis(triphenylphosphine)palladium(0) were loaded into a 100-ml three-necked flask, and the inside of the container was replaced with argon. Further, 26 ml of 1, 2-dimethoxyethane and 12.5 ml (3 eq) of a 2M aqueous solution of sodium carbonate were added, and the whole was refluxed under heat in an oil bath at 90°C for 9 hours. After one night, ion-exchanged water and methylene chloride were added to extract an organic layer, and the layer was washed with ion-exchanged water and a saturated salt solution. The resultant was dried with anhydrous magnesium sulfate, and the solvent was removed by distillation. 4.53 g of a gray solid as a residue were purified by means of silica gel column chromatography (developing solvent: hexane/methylene chloride), and the solvent was removed by distillation, whereby 3.62 g of Compound (H-11) were obtained. The result of the measurement of the FD-MS of obtained Compound (H-11) is shown below.
FD-MS: calcd for $C_{56}H_{37}N_6$ = 780, found m/z = 780 ($M^+$, 100)

Synthesis Example 12 (Synthesis of Compound (H-12))

Compound (H-12) was synthesized as described below.

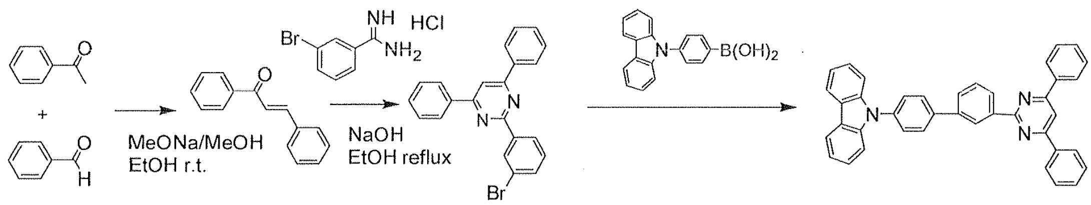


(Intermediate o) (H-12)

10.6 g (100 mmol) of benzaldehyde and 12.0 g (10 mmol) of acetophenone were loaded into a 300-ml three-necked flask, followed by argon replacement. Next, 100 ml of ethanol and 10 ml of a 1N solution of sodium methoxide in methanol were added, and the whole was stirred at room temperature for 5 hours. After that, the temperature of the resultant was increased in an oil bath at 70°C, and the resultant was reacted for an additional 4 hours while ethanol was refluxed. Next, 16. 5 g (70 mmol) of 3-bromobenzamidine hydrochloride and 8.0 g (200 mmol) of sodium hydroxide were added, the temperature of the whole was increased in an oil bath at 70°C, and the whole was reacted for 5 hours. After the completion of the reaction, the precipitated product was separated by filtration and purified by means of silica gel column chromatography (developing solvent: methylene chloride), whereby 13.6 g of (Intermediate o) were obtained (35% yield).
2.71 g (7 mmol) of (Intermediate o), 2.41 g (8.4 mmol) of 4-(N-carbazolyl)phenylboric acid, and 0.291 g (0.25 mmol, 3% Pd) of tetrakis (triphenylphosphine) palladium (0) were loaded into a 100-ml three-necked flask, and the inside of the container was replaced with argon. Further, 26 ml of 1,2-dimethoxyethane and 12.5 ml (3 eq) of a 2M aqueous solution of sodium carbonate were added, and the whole was refluxed under heat in an oil bath at 90°C for 9 hours . After one night, ion-exchanged water and methylene chloride were added to extract an organic layer, and the layer was washed with ion-exchanged water and a saturated salt solution. The resultant was dried with anhydrous magnesium sulfate, and the solvent was removed by distillation. 4.33 g of a gray solid as a residue were purified by means of silica gel column chromatography (developing solvent: hexane/methylene chloride), and the solvent was removed by distillation, whereby 3.52 g of Compound (H-12) were obtained. The result of the measurement of the FD-MS of obtained Compound (H-12) is shown below.
FD-MS: calcd for $C_{40}H_{27}N_3$ = 549.66, found m/z = 549 ($M^+$, 100)

Synthesis Example 13 (Synthesis of Compound (H-13))

Compound (H-13) was synthesized as described below.

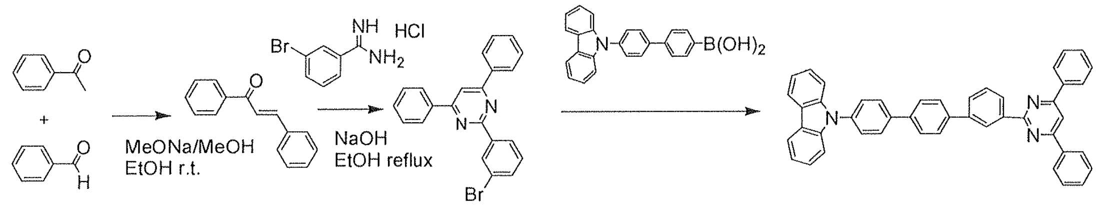


(Intermediate o) (H-13)

Intermediate o was synthesized in the same manner as in Synthesis Example 12. Next, 2.71 g (7 mmol) of (Intermediate o), 3.05 g (8.4 mmol) of 4-carbazol-9-yl-biphenyl-4-boronic acid, and 0.291 g (0.25 mmol, 3% Pd) of tetrakis (triphenylphosphine) palladium (0) were loaded into a 100-ml three-necked flask, and the inside of the container was replaced with argon. Further, 26 ml of 1,2-dimethoxyethane and 12.5 ml (3 eq) of a 2M aqueous solution of sodium carbonate were added, and the whole was refluxed under heat in an oil bath at 90°C for 9 hours. After one night, ion-exchanged water and methylene chloride were added to extract an organic layer, and the layer was washed with ion-exchanged water and a saturated salt solution. The resultant was dried with anhydrous magnesium sulfate, and the solvent was removed by distillation. 4.01 g of a gray solid as a residue were purified by means of silica gel column chromatography (developing solvent: hexane/methylene chloride), and the solvent was removed by distillation, whereby 3.32 g of Compound (H-13) were obtained.
The result of the measurement of the FD-MS of obtained Compound (H-13) is shown below.
FD-MS: calcd for $C_{46}H_{31}N_3$ = 625.76, found m/z = 626 ($M^+$, 100)

Synthesis Example 14 (Synthesis of Compound (H-14))

Compound (H-14) was synthesized as described below.

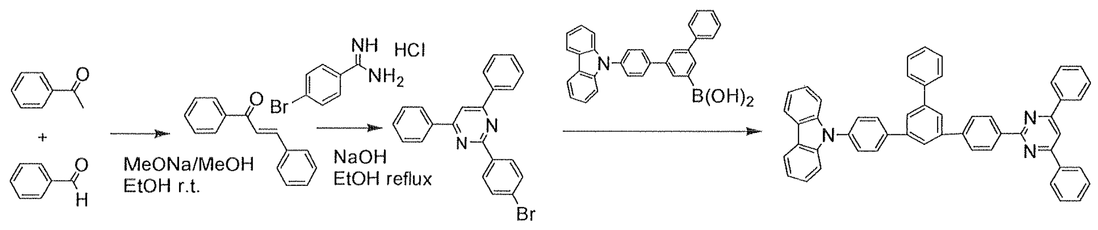


(Intermediate p) (H-14)

10.6 g (100 mmol) of benzaldehyde and 12.0 g (10 mmol) of acetophenone were loaded into a 300-ml three-necked flask, followed by argon replacement. Next, 100 ml of ethanol and 10.0 ml of a 1N solution of sodium methoxide in methanol were added, and the whole was stirred at room temperature for 5 hours. After that, the temperature of the resultant was increased in an oil bath at 70°C, and the resultant was reacted for an additional 4 hours while ethanol was refluxed. Next, 16.5 g (70 mmol) of 4-bromobenzamidine hydrochloride and 8.0 g (200 mmol) of sodium hydroxide were added, the temperature of the whole was increased in an oil bath at 70°C, and the whole was reacted for 5 hours. After the completion of the reaction, the precipitated product was separated by filtration and purified by means of silica gel column chromatography (developing solvent: methylene chloride), whereby 14.0 g of (Intermediate p) were obtained (36% yield).
2.71 g (7 mmol) of (Intermediate p), 3.69 g (8.4 mmol) of 4-carbazol-9-ly-biphenyl-3-phenyl-3-boronic acid, and 0.291 g (0.25 mmol, 3% Pd) of tetrakis (triphenylphosphine) palladium (0) were loaded into a 100-ml three-necked flask, and

the inside of the container was replaced with argon. Further, 26 ml of 1,2-dimethoxyethane and 12.5 ml (3 eq) of a 2M aqueous solution of sodium carbonate were added, and the whole was refluxed under heat in an oil bath at 90°C for 9 hours. After one night, ion-exchanged water and methylene chloride were added to extract an organic layer, and the layer was washed with ion-exchanged water and a saturated salt solution. The resultant was dried with anhydrous magnesium sulfate, and the solvent was removed by distillation. 4.57 g of a gray solid as a residue were purified by means of silica gel column chromatography (developing solvent: hexane/methylene chloride), and the solvent was removed by distillation, whereby 3 . 99 g of Compound (H-14) were obtained. The result of the measurement of the FD-MS of obtained Compound (H-14) is shown below.
FD-MS: calcd for $C_{52}H_{35}N_3$ =701.85, found m/z = 702 ($M^+$, 100)

Synthesis Example 15 (Synthesis of Compound (H-15))

Compound (H-15) was synthesized as described below.

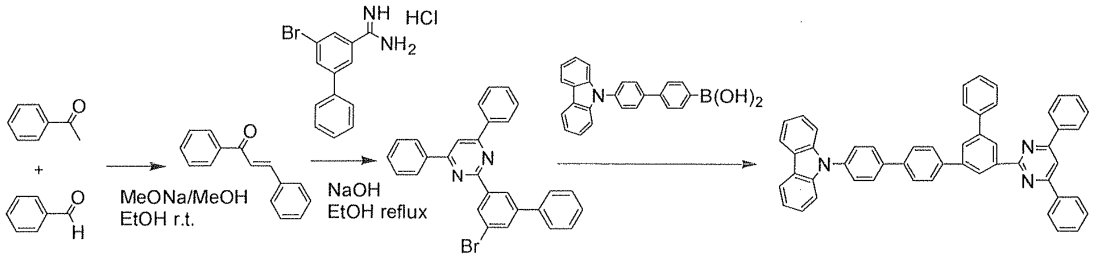


(Intermediate q) (H-15)

10. 6 g (100 mmol) of benzaldehyde and 12.0 g (10 mmol) of acetophenone were loaded into a 300-ml three-necked flask, followed by argon replacement. Next, 100 ml of ethanol and 10.0 ml of a 1N solution of sodium methoxide in methanol were added, and the whole was stirred at room temperature for 5 hours. After that, the temperature of the resultant was increased in an oil bath at 70°C, and the resultant was reacted for an additional 4 hours while ethanol was refluxed. Next, 21.8 g (70 mmol) of 3-phenyl-3-bromobenzamidine hydrochloride and 8.0 g (200 mmol) of sodium hydroxide were added, the temperature of the whole was increased in an oil bath at 70°C, and the whole was reacted for 5 hours. After the completion of the reaction, the precipitated product was separated by filtration and purified by means of silica gel column chromatography (developing solvent: methylene chloride), whereby 14.0 g of (Intermediate q) were obtained (36% yield).
2.18 g (7 mmol) of (Intermediate q), 3.05 g (8.4 mmol) of 4-carbazol-9-ly-biphenyl-4-boronic acid, and 0.291 g (0.25 mmol, 3% Pd) of tetrakis (triphenylphosphine) palladium (0) were loaded into a 100-ml three-necked flask, and the inside of the container was replaced with argon. Further, 26 ml of 1,2 -dimethoxyethane and 12. 5 ml (3 eq) of a 2M aqueous solution of sodium carbonate were added, and the whole was refluxed under heat in an oil bath at 90°C for 9 hours . Afteronenight, ion-exchangedwaterandmethylene chloride were added to extract an organic layer, and the layer was washed with ion-exchanged water and a saturated salt solution. The resultant was dried with anhydrous magnesium sulfate, and the solvent was removed by distillation. 4.45 g of a gray solid as a residue were purified by means of silica gel column chromatography (developing solvent: hexane/methylene chloride), and the solvent was removed by distillation, whereby 3.80 g of Compound (H-15) were obtained. The result of the measurement of the FD-MS of obtained Compound (H-15) is shown below.
FD-MS: calcd for $C_{52}H_{35}N_3$ = 701.85, found m/z = 702 ($M^+$, 100)

Example 1

A glass substrate equipped with an ITO transparent electrode measuring 25 mm in width by 75 mm in length by 0.7 mm in thickness was subjected to ultrasonic cleaning in isopropyl alcohol for 5 minutes. After that, the substrate was subjected to UV ozone cleaning for 30 minutes. The glass substrate equipped with the transparent electrode after the cleaning was mounted on a substrate holder of a vacuum deposition device. First, a copper phthalocyanine film to be described below (hereinafter abbreviated as "CuPc film") having a thickness of 10 nm was formed on the surface

where the transparent electrode was formed in such a manner that the film would cover the transparent electrode. The CuPc film functions as a hole injecting layer. A 4,4'-bis[N-(1-naphthyl)-N-phenylamino]biphenyl film (hereinafter abbreviated as "α-NPD film") to be described below having a thickness of 30 nm was formed on the CuPc film. The α-NPD film functions as a hole transporting layer. Further, Compound (H-1) described above having a thickness of 30 nm as a host material and was deposited from the vapor to form a light emitting layer. At the same time, tris(2-phenylpyridine)Ir (hereinafter abbreviated as "$Ir(ppy)_3$") to be described below as a phosphorescent Ir metal complex dopant were added with each other. The concentration of $Ir(ppy)_3$ in the light emitting layer was set to 5 wt%. The film functions as a light emitting layer. (1,1'-bisphenyl)-4-olato)bis(2-methyl-8-quinolinolato)aluminum was formed into a film having a thickness of 10 nm (hereinafter abbreviated as "BAlq film") on the film. The BAlq film functions as a hole blocking layer. Further, an aluminum complex of 8-hydroxyquinoline was formed into a film having a thickness of 40 nm (hereinafter abbreviated as "Alq film") on the film. The Alq film functions as an electron injecting layer. After that, LiF as an alkali metal halide was deposited from the vapor to have a thickness of 0.2 nm, and then aluminum was deposited from the vapor to have a thickness of 150 nm. The Al/LiF functions as a cathode. Thus, an organic EL device was produced.

The resultant device was subjected to a current test. As a result, green light having an emission luminance of 101 $cd/m^2$ was emitted at a voltage of 5.4 V and a current density of 0.25 $mA/cm^2$. Chromaticity coordinates were (0.32, 0.61), and a current efficiency was 40.4 cd/A. In addition, the device was driven at a constant current and an initial luminance of 5, 000 $cd/m^2$. The time required for the initial luminance to reduce in half to an emission luminance of 2,500 $cd/m^2$ was 624 hours. Table 1 shows the results.

Further, a heat resistance test was performed. That is, the device was subjected to a current test under an environmental condition of 105°C. As a result, it was confirmed that green light having a sufficient emission luminance was emitted even after the lapse of 500 hours from the electricity passing.

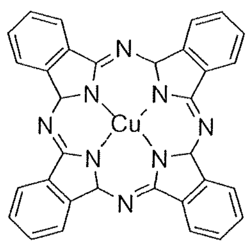

CuPc

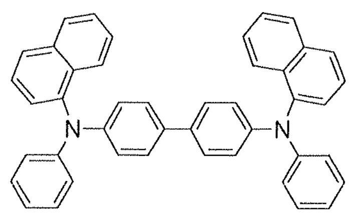

α-NPD

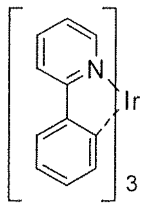

Ir(ppy)3

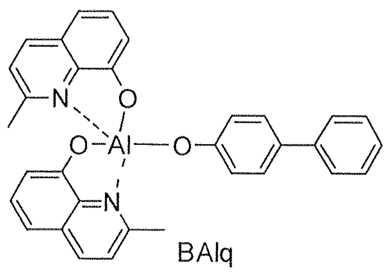

BAlq

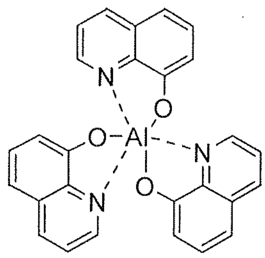

Alq

Examples 2 to 11

Organic EL devices were each produced in the same manner as in Example 1 except that a compound described in Table 1 was used instead of Compound (H-1) as a host material for a light emitting layer, and were each subjected to a current test. Table 1 shows the results.

In addition, a heat resistance test was performed for each of the devices obtained in Examples 2 to 11. That is, each of the devices was subjected to a current test under an environmental condition of 105°C. As a result, it was confirmed that green light having a sufficient emission luminance was emitted even after the lapse of 500 hours from the electricity passing.

Comparative Examples 1 to 4

Organic EL devices were each produced in the same manner as in Example 1 except that a following compound

described in Table 1 was used instead of Compound (H-1) as a host material for a light emitting layer, and were each subjected to a current test. Table 1 shows the results.
In addition, a heat resistance test was performed for each of the devices obtained in Comparative Examples 1 to 4. That is, each of the devices was subjected to a current test under an environmental condition of 105°C. As a result, it was confirmed that an emission luminance significantly reduced after the lapse of 500 hours from the electricity passing.

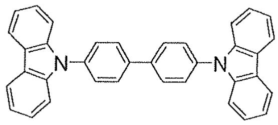

Compound(CBP)

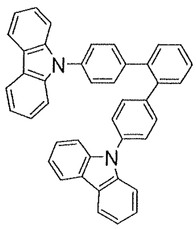

Compound (Comparative 2)

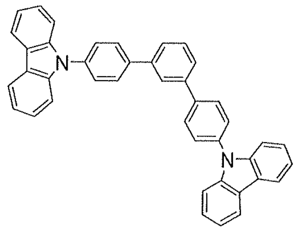

Compound (Comparative 3)

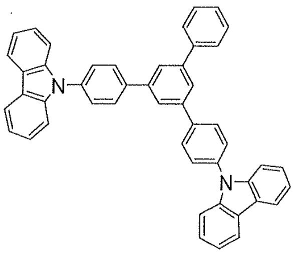

Compound (Comparative 4)

Table 1

| | Host material for light emitting layer | Voltage (V) | Current density (mA/cm$^2$) | Luminance (cd/m$^2$) | Current efficiency (cd/A) | Chromaticity coordinates (x, y) | Luminance half lifetime (hours) Initial luminance 5000 (cd/m$^2$) |
|---|---|---|---|---|---|---|---|
| Example 1 | H-1 | 5.4 | 0.25 | 101 | 40.4 | (0.32, 0.61) | 624 |
| Example 2 | H-2 | 5.5 | 0.26 | 104 | 40.0 | (0.32, 0.61) | 848 |
| Example 3 | H-3 | 5.4 | 0.25 | 102 | 40.8 | (0.32, 0.61) | 795 |
| Example 4 | H-4 | 5.6 | 0.24 | 99 | 41.3 | (0.32, 0.61) | 899 |
| Example 5 | H-5 | 5.5 | 0.26 | 104 | 40.0 | (0.32, 0.61) | 686 |
| Example 6 | H-6 | 5.6 | 0.24 | 101 | 42.1 | (0.32, 0.61) | 780 |
| Example 7 | H-7 | 5.6 | 0.24 | 99 | 41.3 | (0.32, 0.61) | 897 |
| Example 8 | H-8 | 5.5 | 0.26 | 104 | 40.0 | (0.32, 0.61) | 702 |
| Example 9 | H-9 | 5.4 | 0.25 | 103 | 41.2 | (0.32, 0.61) | 789 |
| Example 10 | H-10 | 5.6 | 0.23 | 102 | 44.3 | (0.32, 0.61) | 803 |
| Example 11 | H-11 | 5.4 | 0.25 | 104 | 41.6 | (0.32, 0.61) | 784 |
| Comparative Example 1 | CBP | 5.5 | 0.32 | 101 | 31.6 | (0.32, 0.61) | 403 |
| Comparative Example 2 | Comparative 2 | 5.4 | 0.32 | 104 | 32.5 | (0.32, 0.61) | 358 |
| Comparative Example 3 | Comparative 3 | 5.5 | 0.31 | 98 | 31.6 | (0.32, 0.61) | 427 |

(continued)

| | Host material for light emitting layer | Voltage (V) | Current density ($mA/cm^2$) | Luminance ($cd/m^2$) | Current efficiency (cd/A) | Chromaticity coordinates (x, y) | Luminance half lifetime (hours) Initial luminance 5000 ($cd/m^2$) |
|---|---|---|---|---|---|---|---|
| Comparative Example 4 | Comparative 4 | 5.5 | 0.30 | 102 | 34.0 | (0.33, 0.61) | 426 |
| As shown in Table 1, each of the organic EL devices of Examples 1 to 11 had a high current efficiency and a long lifetime, and emitted green light with high thermostability. | | | | | | | |

Example 12

An organic EL device was produced in the same manner as in Example 1 except that bis(2-benzothienylpyridine) acetylacetonatoiridium (hereinafter abbreviated as "$Ir(btp)_2(acac)$") shown below was added instead of $Ir(ppy)_3$ as a phosphorescent Ir metal complex dopant for a light emitting layer.
The resultant device was subjected to a current test. As a result, red light having an emission luminance of 101 $cd/m^2$ was emitted at a voltage of 7. 3 V and a current density of 1. 2 $mA/cm^2$. Chromaticity coordinates were (0.66, 0.32), and a current efficiency was 8.4 cd/A. In addition, the device was driven at a constant current and an initial luminance of 500 $cd/m^2$. The time required for the initial luminance to reduce in half to an emission luminance of 250 $cd/m^2$ was 1731 hours. Table 2 shows the results.
Further, a heat resistance test was performed. That is, the device was subjected to a current test under an environmental condition of 105°C. As a result, it was confirmed that red light having a sufficient emission luminance was emitted even after the lapse of 500 hours from the electricity passing.

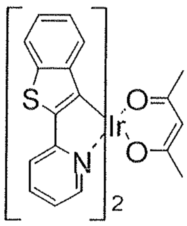

$Ir(btp)_2(acac)$

Examples 13 and 14

Organic EL devices were each produced in the same manner as in Example 12 except that a compound described in Table 2 was used instead of Compound (H-1) as a host material for a light emitting layer, and were each subjected to a current test. Table 2 shows the results.
In addition, a heat resistance test was performed for each of the devices obtained in Examples 13 and 14. That is, each of the devices was subjected to a current test under an environmental condition of 105°C. As a result, it was confirmed that red light having a sufficient emission luminance was emitted even after the lapse of 500 hours from the electricity passing.

Comparative Examples 5 to 7

Organic EL devices were each produced in the same manner as in Example 12 except that any one of the following compounds described in Table 2 was used instead of Compound (H-1) as a host material for a light emitting

layer, and were each subjected to a current test. Table 2 shows the results.
In addition, a heat resistance test was performed for each of the devices obtained in Comparative Examples 5 to 7. That is, each of the devices was subjected to a current test under an environmental condition of 105°C. As a result, it was confirmed that an emission luminance significantly reduced after the lapse of 500 hours from the electricity passing.
Table 2

| | Host material for light emitting layer | Voltage (V) | Current density ($mA/cm^2$) | Luminance ($cd/m^2$) | Current efficiency (cd/A) | Chromaticity coordinates (x,y) | Luminance half lifetime (hours) Initial luminance 500 ($cd/m^2$) |
|---|---|---|---|---|---|---|---|
| Example 12 | H-1 | 7.3 | 1.2 | 101 | 8.4 | (0.66, 0.32) | 1731 |
| Example 13 | H-6 | 7.3 | 1.3 | 102 | 7.8 | (0.66, 0.33) | 1689 |
| Example 14 | H-7 | 7.3 | 1.3 | 101 | 7.8 | (0.66, 0.32) | 1713 |
| Comparative Example 5 | CBP | 7.2 | 4.3 | 98 | 2.3 | (0.65, 0.33) | 451 |
| Comparative Example 6 | Comparative 3 | 7.2 | 3.4 | 103 | 3.0 | (0.66, 0.32) | 769 |
| Comparative Example 7 | Comparative 4 | 7.3 | 3.1 | 100 | 3.2 | (0.66, 0.32) | 842 |
| As shown in Table 2, each of the organic EL devices of Examples 12 to 14 had a high current efficiency and a long lifetime, and emitted red light with high thermostability. | | | | | | | |

Examples 15 to 18

Organic EL devices were each produced in the same manner as in Example 1 except that: tris(2-phenylisoquinoline)iridium (hereinafter abbreviated as "Ir $(piq)_3$") was used instead of Ir $(ppy)_3$ as a phosphorescent Ir metal complex dopant for a light emitting layer; and a compound described in Table 3 was used instead of Compound (H-1) as a host material for the light emitting layer, and were each subjected to a current test. In addition, each of the devices was driven at a constant current and an initial luminance of 1,000 $cd/m^2$, and the time required for the initial luminance to reduce in half to an emission luminance of 500 $cd/m^2$ was measured. Table 3 shows the results.

Examples 19 to 22

Organic EL devices were each produced in the same manner as in each of Examples 15 to 18 except that bis (2-phenylisoquinoline)iridium acetylacetonato ($Ir(piq)_2(acac)$) was used instead of $Ir(piq)_3$ as a phosphorescent Ir metal complex dopant for a light emitting layer, and were each subjected to a current test. In addition, each of the devices was driven at a constant current and an initial luminance of 1, 000 $cd/m^2$, and the time required for the initial luminance to reduce in half to an emission luminance of 500 $cd/m^2$ was measured. Table 3 shows the results.
[Table 3]

Table 3

| | Host material for light emitting layer | Voltage (V) | Current density ($mA/cm^2$) | Luminance ($cd/m^2$) | Current efficiency (cd/A) | Chromaticity coordinates (x,y) | Luminance half lifetime (hours) Initial luminance 1000 ($cd/m^2$) |
|---|---|---|---|---|---|---|---|
| Example 15 | H-11 | 5.5 | 1.3 | 105.0 | 8.1 | (0.67, 0.33) | 20400 |
| Example 16 | H-12 | 5.8 | 1.2 | 103.2 | 8.6 | (0.67, 0.33) | 18500 |
| Example 17 | H-13 | 5.4 | 1.2 | 103.4 | 8.6 | (0.66, 0.33) | 19200 |
| Example 18 | H-14 | 5.6 | 1.2 | 100.5 | 8.4 | (0.67, 0.32) | 17300 |
| Example 19 | H-11 | 5.6 | 1.3 | 103.0 | 7.9 | (0.68, 0.32) | 21800 |
| Example 20 | H-12 | 6.1 | 1.6 | 102.0 | 6.4 | (0.68, 0.32) | 18700 |
| Example 21 | H-13 | 5.3 | 1.2 | 98.0 | 8.19 | (0.68, 0.32) | 18100 |
| Example 22 | H-14 | 5.7 | 1.3 | 103.0 | 7.9 | (0.67, 0.33) | 19100 |

Industrial Applicability

As described above in detail, the organic EL device in which the material for the EL device of the present invention is used is extremely practical because it has high luminous efficiency, high thermostability, and a long lifetime. Therefore, the device is extremely practical and useful as a full-color display, an information display instrument, an on-vehicle display instrument, or a lighting apparatus.

**Claims**

**1.** A material for an organic electroluminescence device comprising a compound represented by the following general formula (1) :

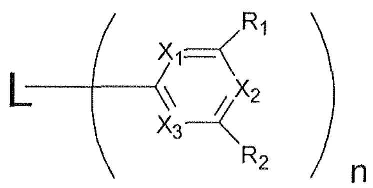

(1)

where:

L represents a linking group having at least one meta bond;
$R_1$ and $R_2$ each independently represent a hydrogen atom, an alkyl group which has 1 to 50 carbon atoms and which may have a substituent, a heterocyclic group which has 5 to 50 ring atoms and which may have a

substituent, an alkoxy group which has 1 to 50 carbon atoms and which may have a substituent, an aryloxy group which has 5 to 50 ring carbon atoms and which may have a substituent, an aralkyl group which has 7 to 50 ring carbon atoms and which may have a substituent, an alkenyl group which has 2 to 50 carbon atoms and which may have a substituent, an alkylamino group which has 1 to 50 carbon atoms and which may have a substituent, an arylamino group which has 5 to 50 ring carbon atoms and which may have a substituent, an aralkylamino group which has 7 to 50 ring carbon atoms and which may have a substituent, an aryl group which has 6 to 50 ring carbon atoms and which may have a substituent, or a cyano group;

$X_1$ to $X_3$ each independently represent =CR- or =N-, at least one of $X_1$ to $X_3$ representing =N- where R represents an aryl group which has 6 to 50 ring carbon atoms and which may have a substituent, a heterocyclic group which has 5 to 50 ring atoms and which may have a substituent, an alkyl group which has 1 to 50 carbon atoms and which may have a substituent, an alkoxy group which has 1 to 50 carbon atoms and which may have a substituent, an aralkyl group which has 7 to 50 ring carbon atoms and which may have a substituent, an aryloxy group which has 5 to 50 ring carbon atoms and which may have a substituent, an arylthio group which has 5 to 50 ring carbon atoms and which may have a substituent, a carboxyl group, a halogen atom, a cyano group, a nitro group, or a hydroxyl group; and

n represents an integer of 1 to 5.

**2.** A material for an organic electroluminescence device according to claim 1, wherein L in the general formula (1) is represented by the following general formula (2):

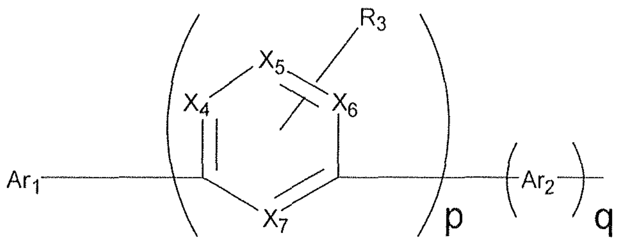

(2)

where:

$X_4$ to $X_7$ each independently represent =CR- or =N- where R represents any one of the same groups as those described above;

$R_3$ represents a hydrogen atom, an alkyl group which has 1 to 50 carbon atoms and which may have a substituent, a heterocyclic group which has 5 to 50 ring atoms and which may have a substituent, an alkoxy group which has 1 to 50 carbon atoms and which may have a substituent, an aryloxy group which has 5 to 50 ring carbon atoms and which may have a substituent, an aralkyl group which has 7 to 50 ring carbon atoms and which may have a substituent, an alkenyl group which has 2 to 50 carbon atoms and which may have a substituent, an alkylamino group which has 1 to 50 carbon atoms and which may have a substituent, an arylamino group which has 5 to 50 ring carbon atoms and which may have a substituent, an aralkylamino group which has 7 to 50 ring carbon atoms and which may have a substituent, an aryl group which has 6 to 50 ring carbon atoms and which may have a substituent, or a cyano group, and two or more $R_3$s may be included;

$Ar_1$ represents a heterocyclic group which has 5 to 50 ring atoms and which may have a substituent, an aryloxy group or aryleneoxy group which has 5 to 50 ring carbon atoms and which may have a substituent, an arylamino group or aryleneamino group which has 5 to 50 ring carbon atoms and which may have a substituent, or an aryl group or arylene group which has 6 to 50 ring carbon atoms and which may have a substituent;

$Ar_2$ represents a heterocyclic group which has 5 to 50 ring atoms and which may have a substituent, an aryleneoxy group which has 5 to 50 ring carbon atoms and which may have a substituent, an aryleneamino group which has 5 to 50 ring carbon atoms and which may have a substituent, or an arylene group which has 6 to 50 ring carbon atoms and which may have a substituent; and

p represents an integer of 1 to 20 and q represents an integer of 1 to 20.

**3.** A material for an organic electroluminescence device according to claim 2, wherein $Ar_1$ has a substituent represented by any one of the following general formulae (3) to (8):

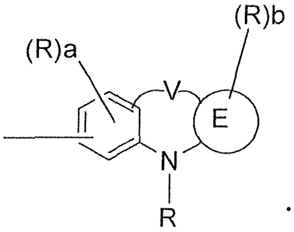 ···(3) 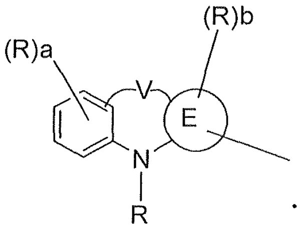 ···(4) 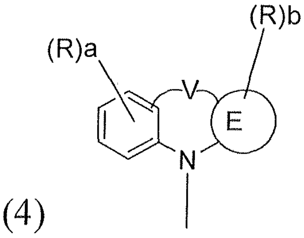 ···(5)

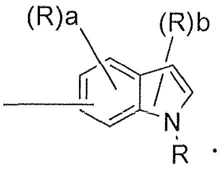 ···(6) 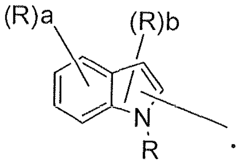 ···(7) 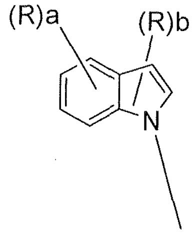 ···(8)

where:

R represents any one of the same groups as those described above, and when two or more Rs are included, Rs may bond to each other to form a ring structure, and a and b each represent an integer of 0 to 4;
V represents a single bond, $-CR_0R_0'-$, $-SiR_0R_0'-$, -O-, -CO-, or $-NR_0$ where $R_0$ and $R_0'$ each independently represent a hydrogen atom, an aryl group which has 6 to 50 ring carbon atoms and which may have a substituent, a heterocyclic group which has 5 to 50 ring atoms and which may have a substituent, or an alkyl group which has 1 to 50 carbon atoms and which may have a substituent, and
E represents a cyclic structure represented by a circle surrounding a symbol E, and represents a cycloalkane residue which has 3 to 20 ring carbon atoms and which may have a substituent, and a carbon atom of which may be substituted by a nitrogen atom, an aromatic hydrocarbon residue which has 4 to 50 ring carbon atoms and which may have a substituent, or a heterocyclic residue which has 4 to 50 ring atoms and which may have a substituent;

**4.** A material for an organic electroluminescence device according to claim 1, wherein L in the general formula (1) is represented by the following general formula (9):

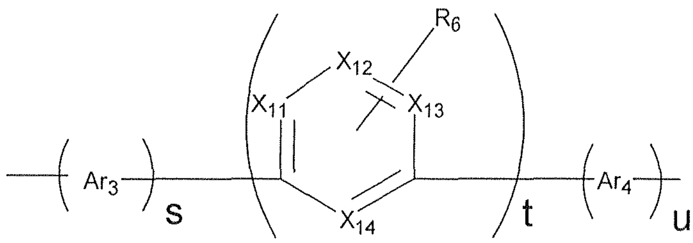 (9)

where:

$X_{11}$ to $X_{14}$ each independently represent =CR- or =N- where R represents any one of the same groups as those described above;
$R_6$ represents a hydrogen atom, an alkyl group which has 1 to 50 carbon atoms and which may have a substituent, a heterocyclic group which has 5 to 50 ring atoms and which may have a substituent, an alkoxy group which has 1 to 50 carbon atoms and which may have a substituent, an aryloxy group which has 5 to 50 ring carbon atoms and which may have a substituent, an aralkyl group which has 7 to 50 ring carbon atoms and which may have a substituent, an alkenyl group which has 2 to 50 carbon atoms and which may have a substituent, an

alkylamino group which has 1 to 50 carbon atoms and which may have a substituent, an arylamino group which has 5 to 50 ring carbon atoms and which may have a substituent, an aralkylamino group which has 7 to 50 ring carbon atoms and which may have a substituent, an aryl group which has 6 to 50 ring carbon atoms and which may have a substituent, or a cyano group, and two or more $R_6$s may be included;
$Ar_3$ and $Ar_4$ each independently represent a heterocyclic group which has 5 to 50 ring atoms and which may have a substituent, an aryleneoxy group which has 5 to 50 ring carbon atoms and which may have a substituent, an aryleneamino group which has 5 to 50 ring carbon atoms and which may have a substituent, or an arylene group which has 6 to 50 ring carbon atoms and which may have a substituent; and
s represents an integer of 0 to 20, t represents an integer of 1 to 20, and u represents an integer of 0 to 20.

**5.** A material for an organic electroluminescence device according to claim 1, wherein L in the general formula (1) is represented by the following general formula (10):

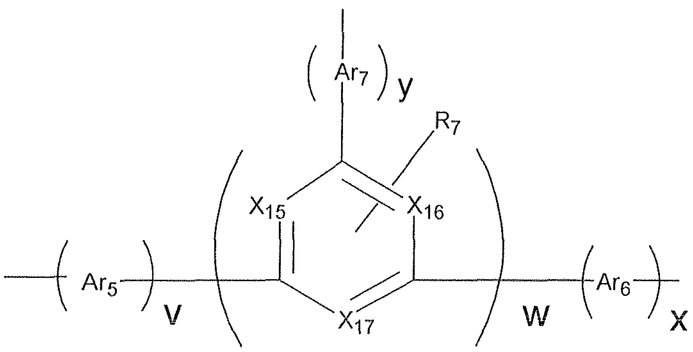
(10)

where:

$X_{15}$ to $X_{17}$ each independently represent =CR- or =N- where R represents any one of the same groups as those described above;
$R_7$ represents a hydrogen atom, an alkyl group which has 1 to 50 carbon atoms and which may have a substituent, a heterocyclic group which has 5 to 50 ring atoms and which may have a substituent, an alkoxy group which has 1 to 50 carbon atoms and which may have a substituent, an aryloxy group which has 5 to 50 ring carbon atoms and which may have a substituent, an aralkyl group which has 7 to 50 ring carbon atoms and which may have a substituent, an alkenyl group which has 2 to 50 carbon atoms and which may have a substituent, an alkylamino group which has 1 to 50 carbon atoms and which may have a substituent, an arylamino group which has 5 to 50 ring carbon atoms and which may have a substituent, an aralkylamino group which has 7 to 50 ring carbon atoms and which may have a substituent, an aryl group which has 6 to 50 ring carbon atoms and which may have a substituent, or a cyano group, and two or more $R_7$s may be included;
$Ar_5$ to $Ar_7$ each independently represent a heterocyclic group which has 5 to 50 ring atoms and which may have a substituent, an aryleneoxy group which has 5 to 50 ring carbon atoms and which may have a substituent, an aryleneamino group which has 5 to 50 ring carbon atoms and which may have a substituent, or an arylene group which has 6 to 50 ring carbon atoms and which may have a substituent;
v represents an integer of 0 to 20, w represents an integer of 1 to 20, x represents an integer 0 to 20, and y represents an integer of 0 to 20.

**6.** A material for an organic electroluminescence device according to claim 4, wherein the material has at least one substituent represented by any one of the following general formulae (3) to (8):

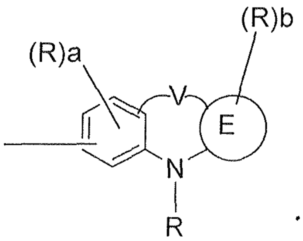 ···(3) 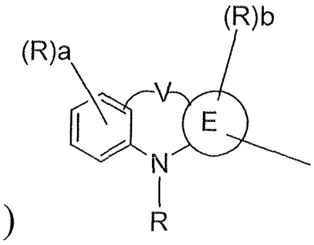 ···(4) 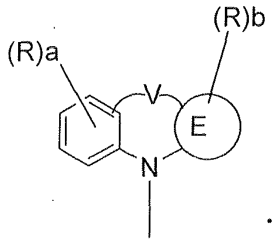 ···(5)

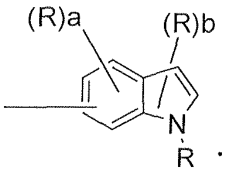 ···(6) 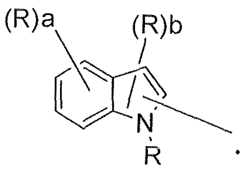 ···(7) 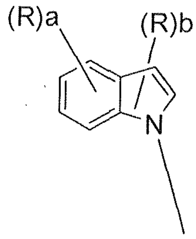 ···(8)

where:

R represents any one of the same groups as those described above, and when two or more Rs are included, Rs may bond to each other to form a ring structure, and a and b each represent an integer of 0 to 4;
V represents a single bond, $-CR_0R_0'-$, $-SiR_0R_0'-$, -O- -CO-, or $-NR_0$ where $R_0$ and $R_0'$ each independently represent a hydrogen atom, an aryl group which has 6 to 50 ring carbon atoms and which may have a substituent, a heterocyclic group which has 5 to 50 ring atoms and which may have a substituent, or an alkyl group which has 1 to 50 carbon atoms and which may have a substituent, and
E represents a cyclic structure represented by a circle surrounding a symbol E, and represents a cycloalkane residue which has 3 to 20 ring carbon atoms and which may have a substituent, and a carbon atom of which may be substituted by a nitrogen atom, an aromatic hydrocarbon residue which has 4 to 50 ring carbon atoms and which may have a substituent, or a heterocyclic residue which has 4 to 50 ring atoms and which may have a substituent;

**7.** A material for an organic electroluminescence device according to claim 5, wherein the material has at least one substituent represented by any one of the following general formulae (3) to (8):

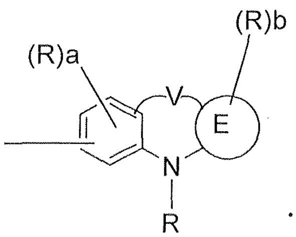 ···(3) 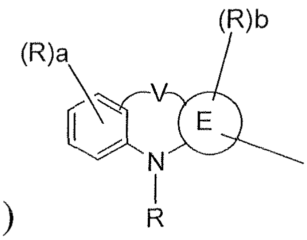 ···(4) 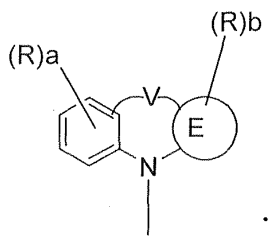 ···(5)

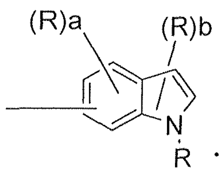 ···(6) 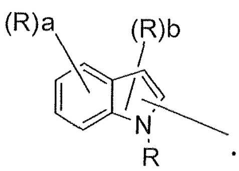 ···(7) 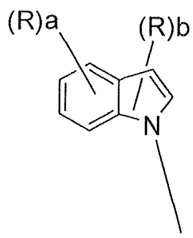 ···(8)

where:

R represents any one of the same groups as those described above, and when two or more Rs are included, Rs may bond to each other to form a ring structure, and a and b each represent an integer of 0 to 4;
V represents a single bond, $-CR_0R_0'-$, $-SiR_0R_0'-$, -O-, -CO-, or $-NR_0$ where $R_0$ and $R_0'$ each independently represent a hydrogen atom, an aryl group which has 6 to 50 ring carbon atoms and which may have a substituent, a heterocyclic group which has 5 to 50 ring atoms and which may have a substituent, or an alkyl group which has 1 to 50 carbon atoms and which may have a substituent, and
E represents a cyclic structure represented by a circle surrounding a symbol E, and represents a cycloalkane residue which has 3 to 20 ring carbon atoms and which may have a substituent, and a carbon atom of which may be substituted by a nitrogen atom, an aromatic hydrocarbon residue which has 4 to 50 ring carbon atoms and which may have a substituent, or a heterocyclic residue which has 4 to 50 ring atoms and which may have a substituent;

**8.** A material for an organic electroluminescence device according to any one of claims 1 to 7, wherein the material comprises a host material- in a light emitting layer of an organic electroluminescence device.

**9.** An organic electroluminescence device comprising an organic thin film layer composed of one or more layers including at least a light emitting layer, the organic thin film layer being interposed between a cathode and an anode, wherein at least one layer of the organic thin film layer contains the material for an organic electroluminescence device according to any one of claims 1 to 7.

**10.** An organic electroluminescence device according to claim 9, wherein the light emitting layer contains a host material and a phosphorescent material, and the host material contains the material for an organic electroluminescence device according to any one of claims 1 to 7.

**11.** An organic electroluminescence device according to claim 9, wherein a reducing dopant is added to an interfacial region between the cathode and the organic thin film layer.

## INTERNATIONAL SEARCH REPORT

International application No.
PCT/JP2005/003783

A. CLASSIFICATION OF SUBJECT MATTER
Int.Cl7 C09K11/06, C07D239/26, 401/14, 403/10, H05B33/14

According to International Patent Classification (IPC) or to both national classification and IPC

B. FIELDS SEARCHED

Minimum documentation searched (classification system followed by classification symbols)
Int.Cl7 C09K11/06, C07D239/26, 401/14, 403/10, H05B33/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 2004-22334 A (Konica Minolta Holdings Kabushiki Kaisha), 22 January, 2004 (22.01.04), Par. Nos. [0049] to [0060], [0099] to [0118] (Family: none) | 1,8-11 |
| X | JP 2003-22893 A (Fuji Photo Film Co., Ltd.), 24 January, 2003 (24.01.03), Par. Nos. [0063] to [0072], [0097] to [0098] (Family: none) | 1,9 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 June, 2005 (16.06.05) | 09 August, 2005 (09.08.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

INTERNATIONAL SEARCH REPORT

International application No.
PCT/JP2005/003783

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 2-7
because they relate to parts of the international application that do not comply with the prescribed requirements to such an extent that no meaningful international search can be carried out, specifically:
As all of $R_3$ of Chem. Form. 2 of claims 2-3, $R_6$ of Chem. Form. 4 of claims 4 and 6 and $R_7$ of Chem. Form. 5 of claims 5 and 7 have a constitution of infeasible bonding, the inventions of claims 2-7 are unclear and the prescribed requirements are not satisfied to such an (continued to extra sheet)

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest.
☐ No protest accompanied the payment of additional search fees.

Form PCT/ISA/210 (continuation of first sheet (2)) (January 2004)

INTERNATIONAL SEARCH REPORT

International application No.
PCT/JP2005/003783

Continuation of Box No.II-2 of continuation of first sheet(2)

extent that no meaningful international search can be carried out.

Form PCT/ISA/210 (extra sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 01072927 A


### Non-patent literature cited in the description


- *Jpn. J. Appl. Phys.,* 1999, vol. 38, L1502